(19) Europäisches Patentamt
European Patent Office
Office européen des brevets

(11) EP 0 912 467 B1

(12) **FASCICULE DE BREVET EUROPEEN**

(45) Date de publication et mention de la délivrance du brevet:
**24.04.2002 Bulletin 2002/17**

(21) Numéro de dépôt: **97930607.3**

(22) Date de dépôt: **27.06.1997**

(51) Int Cl.[7]: **C07B 53/00**, C07C 67/31, C07C 209/52

(86) Numéro de dépôt international:
**PCT/FR97/01154**

(87) Numéro de publication internationale:
**WO 98/00375 (08.01.1998 Gazette 1998/01)**

(54) **PROCEDE D'HYDROGENATION ASYMETRIQUE D'UN COMPOSE CETONIQUE ET DERIVE**

VERFAHREN ZUR ASYMMETRISCHEN HYDRIERUNG VON KETONVERBINDUNGEN UND IHREN DERIVATEN

ASYMMETRIC HYDROGENATION METHOD OF A KETONIC COMPOUND AND DERIVATIVE

(84) Etats contractants désignés:
**AT BE CH DE DK ES FI FR GB GR IE IT LI LU MC NL PT SE**

(30) Priorité: **28.06.1996 FR 9608060**

(43) Date de publication de la demande:
**06.05.1999 Bulletin 1999/18**

(73) Titulaire: **RHODIA CHIMIE**
**92408 Courbevoie Cédex (FR)**

(72) Inventeurs:
- **MATHEY, François**
  **F-75004 Paris (FR)**
- **ROBIN, Frédéric**
  **F-92120 Montrouge (FR)**
- **MERCIER, François**
  **F-78000 Versailles (FR)**
- **SPAGNOL, Michel**
  **F-69008 Lyon (FR)**

(74) Mandataire: **Dutruc-Rosset, Marie-Claude et al**
**Rhodia Services,**
**Direction de la Propriété Industrielle,**
**40, rue de la Haie-Coq**
**93306 Aubervilliers Cedex (FR)**

(56) Documents cités:
**EP-A- 0 302 021**
**EP-A- 0 437 690**
**WO-A-95/21151**
**WO-A-96/20202**
**FR-A- 2 693 190**

- **A. BR QUE ET AL: "The use of 1-phosphanorbornadienes with chiral phosphorus at the bridgehead in catalytic asymmetric hydrogenation of dehydroaminoacids" NEW JOURNAL OF CHEMISTRY, vol. 13, no. 4/5, 1989, pages 369-374, XP000618266**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet un procédé d'hydrogénation asymétrique d'un composé cétonique et dérivé.

L'invehtion vise l'utilisation de complexes métalliques optiquement actifs comme catalyseurs d'hydrogénation asymétrique d'un composé cétonique et dérivé.

Différents procédés d'hydrogénation asymétrique sont décrits dans la littérature.

Il est connu selon Fr-A-2693190 d'effectuer l'hydrogénation catalytique énantiosélective de la liaison C=O cétonique en fonction alcool secondaire, en présence d'un composé diallylique de (diphosphino)ruthénium.

Dans EP-A-437690, on a mentionné l'hydrogénation d'un composé carbonylé, en présence d'un complexe de l'iridium et d'un ligand diphosphoré, les atomes de phosphore ne faisant pas partie d'un cycle mais étant portés par une chaîne aliphatique ou bien étant substituants d'un cycle.

Il est décrit dans EP-A-302021, un procédé de préparation d'arylamines optiquement actives par hydrogénation de cétimines N-arylées, en mettant en oeuvre un complexe à base de rhoduim et d'un ligand dénommé α-POOP, DIOP, PHENPHOS, DIPAMP, CHIRAPHOS, PROPHOS, CYCPHOS ou NORPHOS.

Dans WO-A-95/21151, il est décrit un procédé d'hydrogénation d'une imine, en présence d'un catalyseur à l'iridium, le catalyseur pouvant inclure une phosphine, la caractéristique du procédé étant de conduire la réaction dans un milieu comprenant un chlorure, bromure ou iodure d'ammonium ou métallique et éventuellement un acide.

Anne Brèque et al [New Journal of Chem 13, n°4/5, pp.369-374 (1989)] ont décrit l'utilisation de complexes de rhodium et de 1-phosphonorbornadiène pour effectuer l'hydrogénation assymétrique des deshydriaminoacides.

Dans la demande de brevet français n°94/15757 et PCT/FR95/01716, on a décrit de nouvelles diphosphines optiquement actives de bis-[1-phospha-2,3-diphényl-4,5-diméthylnorbornadiène] répondant aux formules suivantes :

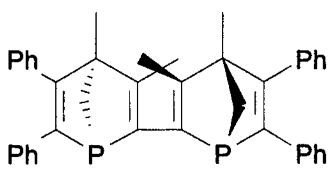

(S,S) - (+)

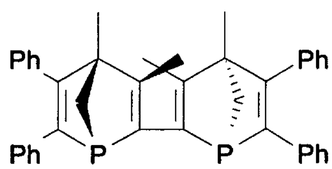

(R,R) - (-)

Les nouvelles phosphines sont obtenues selon un procédé de dédoublement du mélange racémique de bis-[1-phospha-2,3-diphényl-4,5-diméthylnorbornadiène] qui consiste à le faire réagir avec un complexe de palladium ou de platine comme auxiliaire chiral, dans un solvant organique formant ainsi des complexes diastéréoisomères, puis à dédoubler lesdits complexes optiquement purs.

Le mélange des deux diastéroisomères de départ (d'une part un méso et d'autre part un racémique) peut être obtenu selon l'enseignement décrit par F. Mathey et al, dans Bull. Soc. Chim. Fr. 129, pp.1-8 (1992].

Conformément au procédé de l'invention décrit dans FR n°94/15757, on effectue le dédoublement du mélange racémique de bis-[1-phospha-2,3-diphényl-4,5-diméthylnorbornadiène] en le faisant réagir avec un complexe de palladium ou de platine, comme auxiliaire chiral.

On peut faire appel à un complexe de palladium. Ce type d'auxiliaire chiral est largement décrit dans la littérature, notamment par Sei Otsuka et al, dans Journal of the American Chemical Society 93, pp. 4301 (1971).

On peut également faire appel à un complexe de platine et l'on peut se référer plus particulièrement aux travaux de A. C. Cope [Journal of the American Chemical Society 90, pp. 909 (1968)].

Le complexe chiral mis en oeuvre répond plus particulièrement à la formule générale (VII) :

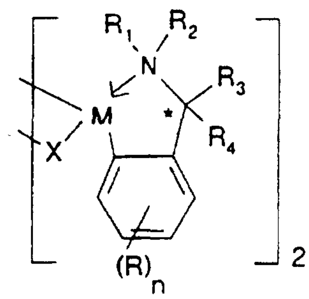

(VII)

dans ladite formule :

- M représente le palladium et/ou le platine,
- $R_1$, $R_2$, $R_3$ et $R_4$ représentent un atome d'hydrogène ou un radical alkyle ayant de 1 à 10 atomes de carbone ou un radical cycloalkyle ayant de 3 à 10 atomes de carbone,
- $R_3$ et $R_4$ sont différents et au moins l'un des deux représente un atome d'hydrogène,
- R a la signification donnée pour $R_1$, $R_2$, $R_3$ et $R_4$,
- X représente un atome d'halogène,
- n est un nombre de 0 à 4,
- lorsque n est supérieur à 1, deux radicaux R et les 2 atomes successifs du cycle benzénique peuvent former entre eux, un cycle ayant de 5 à 7 atomes de carbone.

Plus préférentiellement, le complexe mis en oeuvre répond à la formule précitée dans laquelle $R_1$, $R_2$, $R_3$ et $R_4$ représentent un atome d'hydrogène ou un radical méthyle, X représente un atome de chlore et n est égal à 0.
Lorsque n est égal à 2, deux radicaux R forment un cycle benzénique.
Comme exemples plus spécifiques de complexes de palladium convenant à la présente invention obtenus indifféremment à partir de (R)-(+) ou (S)-(-)-N,N-diméthylphényléthylamine, on peut mentionner :

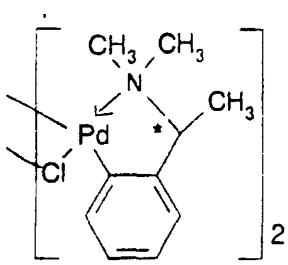

(VII')

La quantité de complexe des métaux précités exprimée en métal est généralement de 0,5 à 1 atome de métal par atome de phosphore.
On fait appel à un solvant organique qui solubilise tous les réactifs. Le solvant doit être inerte vis-à-vis de la diphosphine.
A titre d'exemples non limitatifs de solvants convenant dans le procédé de l'invention, on peut citer :

- les hydrocarbures aliphatiques et plus particulièrement les paraffines tels que notamment, le pentane, l'hexane, l'heptane, l'octane, l'isooctane, le nonane, le décane, l'undécane, le tétradécane, l'éther de pétrole et le cyclohexane ; les hydrocarbures aromatiques comme notamment le benzène, le toluène, les xylènes, l'éthylbenzène, les diéthylbenzènes, les triméthylbenzènes, le cumène, le pseudocumène, les coupes pétrolières constituées d'un mélange d'alkylbenzènes notamment les coupes de type Solvesso®,
- les hydrocarbures halogénés aliphatiques ou aromatiques, et l'on peut mentionner : les hydrocarbures perchlorés tels que notamment le trichlorométhane, le tétrachloroéthylène ; les hydrocarbures partiellement chlorés tels que le dichlorométhane, le dichloroéthane, le tétrachloroéthane, le trichloroéthylène, le 1-chlorobutane, le 1,2-dichlorobutane ; le monochlorobenzène, le 1,2-dichlorobenzène, le 1,3-dichlorobenzène, le 1,4-dichlorobenzène ou des mélanges de différents chlorobenzènes.

Parmi tous ces solvants, le benzène et le toluène sont préférés.

La concentration de la diphosphine dans le solvant réctionnel est de préférence, entre 0,05 et 1 mole/litre et encore plus particulièrement entre 0,05 et 0,2 mole/litre.

La séparation est avantageusement conduite à température ambiante généralement comprise entre 15°C et 25°C.

Elle a lieu de préférence sous atmosphère contrôlée de gaz inertes. On peut établir une atmosphère de gaz rares, de préférence l'argon mais il est plus économique de faire appel à l'azote.

On obtient un mélange de complexes de palladium ou platine et de diphosphine correspondant à chaque énantiomère.

Ils répondent plus particulièrement aux formules suivantes :

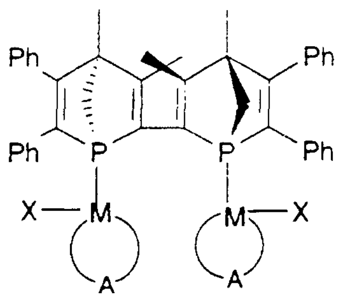

(VIII a)

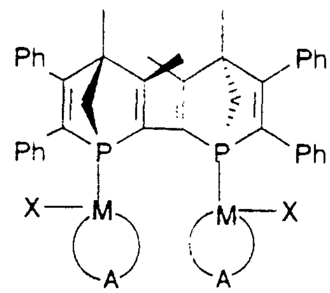

(VIII b)

dans lesdites formules, M représente le palladium ou le platine, X un atome d'halogène, de préférence, le chlore et A symbolise le reste d'un complexe métallique chiral répondant à l'une des formules (VII) et préférentiellement (VII').

Dans une étape suivante, on récupère les deux énantiomères purs.

On concentre par évaporation le solvant puis on effectue la séparation d'une manière connue [A. Bertheillier - Dunod Paris (1972)] par chromatographie liquide sur colonne, avec, de préférence un support en silice.

On élue la colonne avec un mélange de solvants appropriés, de préférence, un mélange toluène/acétate d'éthyle comprenant préférentiellement, 80 % en volume de toluène et 20 % en volume d'acétate d'éthyle.

On récupère les deux énantiomères isolés, purs, sous forme de deux complexes diastéréoisomères ayant les caractéristiques suivantes :

$$RMN^{31}P = \delta(CH_2Cl_2) = 55,9 \text{ ppm}$$

$$RMN^{31}P = \delta(CH_2Cl_2) = 53,6 \text{ ppm}$$

On récupère les deux énantiomères de la diphosphine purs en effectuant la décomplexation.

A cet effet, on utilise notamment un sel de l'acide cyanhydrique, de préférence, un sel alcalin et encore plus préférentiellement le sodium : ledit sel étant mis en solution dans le minimum d'eau nécessaire.

On solubilise les complexes dans un solvant organique tel que, par exemple, le dichlorométhane, puis l'on introduit sous agitation, le sel de l'acide cyanhydrique mis en oeuvre généralement en excès représentant de 2 à 5 moles par atome de métal.

L'opération est également conduite sous atmosphère contrôlée et à température ambiante.

On récupère l'énantiomère dans la phase organique qui est séparée, lavée à l'eau et séchée, par exemple sur sulfate de sodium.

On obtient les deux énantiomères du bis-[1-phospha-2,3-diphényl-4,5-diméthylnorbornadiène], isolés purs répondant aux formules [(Ia) - (S,S) (+)] et [(Ib) - (R,R) (-)] précitées, dont les caractéristiques sont les suivantes :

$$RMN^{31}P = \delta(CDCl_3) = -13,2 \text{ ppm} - [\alpha]_D = +231\ ° \ (c = 1, C_6D_6).$$

$$RMN^{31}P = \delta(CDCl_3) = -13,2 \text{ ppm} - [\alpha]_D = -198° \ (c = 1, C_6D_6)$$

(avec un $[\alpha]_D$ déterminé pour une concentration de 10 mg/ml et à température ambiante).

Poursuivant ses recherches, la demanderesse a trouvé que les nouvelles diphosphines optiquement actives telles que précitées à l'état de complexes métalliques, pouvaient être utilisées comme catalyseurs d'hydrogénation asymétrique de composés cétoniques et dérivés.

Les diphosphines optiquement actives de formule (Ia) ou (Ib) servent de ligands dans la formation de complexes avec des métaux de transition.

Lesdits complexes comprennent une diphosphine optiquement active et un métal de transition qui sont caractérisés par le fait que le ligand répond à l'une des formules suivantes :

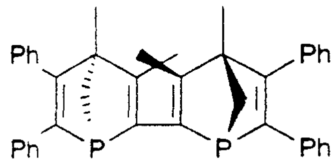

(Ia) - (S,S) - (+)

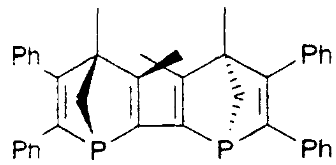

(Ib) - (R,R) - (-)

Comme exemples de métaux de transition capables de former des complexes, on peut citer notamment les métaux tels le rhodium, le ruthénium, le rhénium, l'iridium, le cobalt, le nickel, le platine, le palladium.

Parmi les métaux précités, le rhodium, le ruthénium et l'iridium sont préférés.

Des exemples spécifiques desdits complexes de la présente invention sont donnés ci-après, sans caractère limitatif.

Dans lesdites formules, (P*P) représente la diphosphine de formule (Ia) ou (Ib).

Les complexes de rhodium et d'iridium peuvent être représentés par les formules suivantes :

$$[M\ L_2(P^*P)]\ Y \qquad \text{(IIa)}$$

$$[M\ L_2(P^*P)]\ Y \qquad \text{(IIb)}$$

dans lesdites formules :

- (P*P) représente dans la formule (IIa) la diphosphine de formule (Ia) et dans la formule (IIb) la diphosphine de formule (Ib),
- M représente le rhodium ou l'iridium,
- Y représente un ligand anionique coordinant,
- L représente un ligand neutre.

Les complexes préférés de rhodium ou d'iridium répondent à la formule (IIa) ou (IIb) dans laquelle :

- L représente une oléfine ayant de 2 à 12 atomes de carbone et deux ligands L peuvent être liés entre eux pour former une chaîne hydrocarbonée polyinsaturée, linéaire ou cyclique ; L représentant de préférence, le 1,5-cyclooctadiène, le norbornadiène, l'éthylène,
- Y représente un anion $PF_6^-$, $PCl_6^-$, $BF_4^-$, $BCl_4^-$, $SbF_6^-$, $SbCl_6^-$, $BPh_4^-$, $ClO_4^-$, CN-, $CF_3SO_3^-$, halogène de préférence, $Cl^-$ ou $Br^-$, un anion 1,3-dicétonate, alkylcarboxylate, haloalkylcarboxylate avec un radical alkyle inférieure, un anion phénylcarboxylate ou phénolate dont le cycle benzénique peut être substitué par des radicaux alkyle inférieurs et/ou des atomes d'halogène.

Par radicaux alkyle inférieurs, on entend généralement un radical alkyle linéaire ou ramifié ayant de 1 à 4

atomes de carbone.

D'autres complexes d'iridium peuvent être représentés par les formules :

$$[Ir\ L\ (P^*P)]\ Y \quad (IIIa)$$

$$[Ir\ L\ (P^*P)]\ Y \quad (IIIb)$$

dans lesdites formules, (P*P), L et Y ont les significations données pour les formules (IIa) et (IIb).

Pour ce qui est des complexes de ruthénium, ils répondent préférentiellement aux formules suivantes :

$$[RuY_1Y_2(P^*P)] \quad (IVa)$$

$$[RuY_1Y_2(P^*P)] \quad (IVb)$$

dans lesdites formules :

- (P*P) représente dans la formule (IVa) la diphosphine de formule (Ia) et dans la formule (IVb) la diphosphine de formule (Ib),
- $Y_1$ et $Y_2$, identiques ou différents, représentent de préférence, un anion $PF_6^-$, $PCl_6^-$, $BF_4^-$, $BCl_4^-$, $SbF_6^-$, $SbCl_6^-$, $BPh_4^-$, $ClO_4^-$, $CF_3SO_3^-$, un atome d'halogène, plus particulièrement, chlore ou brome ou un anion carboxylate, préférentiellement, acétate, trifluoroacétate.

D'autres complexes du ruthénium susceptibles d'être mis en oeuvre dans la procédé de l'invention répondent aux formules ci-après :

$$[RuY_1Ar(P^*P)Y_2] \quad (IVc)$$

$$[RuY_1Ar(P^*P)Y_2] \quad (IVd)$$

dans lesdites formules :

- (P*P) représente dans la formule (IVc) la diphosphine de formule (Ia) et dans la formule (IVd) la diphosphine de formule (Ib),
- Ar représente le benzène, le p-méthylisopropylbenzène, l'hexaméthylbenzène,
- $Y_1$ représente un atome d'halogène, de préférence, chlore ou brome,
- $Y_2$ représente un anion de préférence, un anion $PF_6^-$, $PCl_6^-$, $BF_4^-$, $BCl_4^-$, $SbF_6^-$, $SbCl_6^-$, $BPh_4^-$, $ClO_4^-$, $CF_3SO_3^-$.

Il est également possible de mettre en oeuvre dans le procédé de l'invention des complexes à base de palladium et de platine.

Comme exemples plus spécifiques desdits complexes, on peut mentionner entre autres $PdCl_2(P^*P)$ et $PtCl_2(P^*P)$. dans lesquels (P*P) représente la diphosphine de formule (Ia) ou (Ib).

Les complexes comprenant la diphosphine précitée et le métal de transition peuvent être préparés selon les procédés connus décrits dans la littérature.

Pour la préparation des complexes de ruthénium, on peut se référer notamment à la publication de J.-P. Genêt [Acros Organics Acta, 1, Nr. 1, pp. 1-8 (1994)] et pour les autres complexes, à l'article de Schrock R. et Osborn J.A. [Journal of the American Chemical Society, 93, pp. 2397 (1971)].

Ils peuvent être préparés en particulier par réaction de la diphosphine de formule (Ia) ou (Ib) avec le composé de métal de transition, dans un solvant organique approprié.

La réaction est conduite à une température comprise entre la température ambiante (de 15 à 25°C) et la température de reflux du solvant réactionnel.

Comme exemples de solvants organiques, on peut mentionner entre autres, les hydrocarbures aliphatiques,

halogénés ou non et plus particulièrement, l'hexane, l'heptane, l'isooctane, le décane, le benzène, le toluène, le chlorure de méthylène, le chloroforme ; des solvants de type éther ou cétone et notamment le diéthyléther, le tétrahydrofurane, l'acétone, la méthyléthylcétone ; les solvants de type alcool, de préférence, le méthanol ou l'éthanol.

Les complexes métalliques selon l'invention, récupérés selon les techniques classiques (filtration ou cristallisation) sont utilisés dans des réactions d'hydrogénation asymétrique de substrats précisés ci-après.

Il a maintenant été trouvé et c'est ce qui constitue l'objet de la présente invention, un procédé d'hydrogénation asymétrique d'un composé cétonique et dérivé caractérisé par le fait que l'on effectue l'hydrogénation asymétrique dudit composé, en présence d'une quantité efficace d'un complexe métallique comprenant à titre de ligand, la diphosphine optiquement active de formule (Ia) ou (Ib) et un métal de transition.

Le composé cétonique ou dérivé répond plus particulièrement à la formule générale (V):

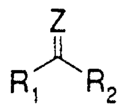
(V)

dans ladite formule (V) :

- $R_1$ est différent de $R_2$,
- $R_1$ et $R_2$ représentent un radical hydrocarboné ayant de 1 à 30 atomes de carbone comprenant éventuellement un ou plusieurs groupes fonctionnels,
- $R_1$ et $R_2$, peuvent former un cycle comprenant éventuellement un autre hétéroatome,
- Z est ou comprend un hétéroatome, oxygène ou azote ou un groupe fonctionnel comprenant au moins un de ces hétéroatomes.

Dans la formule (V), $R_1$ et $R_2$ représentent un radical hydrocarboné monovalent, substitué ou non qui peut être un radical aliphatique acyclique saturé ou insaturé, linéaire ou ramifié ; un radical carbocyclique ou hétérocyclique saturé, insaturé ou aromatique, monocyclique ou polycyclique.

$R_1$ et $R_2$ peuvent prendre diverses significations. Différents exemples sont donnés ci-après mais ils ne sont en aucun cas limitatif.

Dans les composés de formule (V), $R_1$ et $R_2$ représentent un radical aliphatique acyclique, saturé ou insaturé, linéaire ou ramifié.

Plus précisément, $R_1$ et $R_2$ représentent un radical aliphatique acyclique linéaire ou ramifié ayant de préférence de 1 à 12 atomes de carbone, saturé ou comprenant une à plusieurs insaturations sur la chaîne, généralement, 1 à 3 insaturations qui peuvent être des doubles liaisons simples ou conjuguées ou des triples liaisons.

La chaîne hydrocarbonée peut être éventuellement:

- interrompue par l'un des groupes suivants W :

  $$-O-; -CO- ; COO- ; -NT- ; -CO-NT- ; -S- ; -SO_2- ; -NT-CO- ;$$

  dans lesdites formules T représente un atome d'hydrogène, un groupe alkyle linéaire ou ramifié ayant de 1 à 6 atomes de carbone, de préférence, un radical méthyle ou éthyle,
- et/ou porteuse de l'un des substituants suivants :

  $$-OH ; -COOH ; -COOX ; -CO-N(T)(T) ; -COOT ; -CHO ; -COT ; -NO_2 ; -X ; -CF_3.$$

dans ces formules, les radicaux T sont identiques ou différents et X représente un atome d'halogène.

Il est également possible que $R_1$ et $R_2$ représentent un radical aliphatique acyclique, saturé ou insaturé, linéaire ou ramifié éventuellement porteur d'un substituant cyclique. Par cycle, on entend un cycle carbocyclique ou hétérocyclique, saturé, insaturé ou aromatique.

Le radical aliphatique acyclique peut être relié au cycle par un lien valentiel ou par l'un des groupes W précités.

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-mêmes éventuellement porteurs d'un ou plusieurs substituants.

Comme exemples de tels radicaux, on peut mentionner, entre autres, le radical benzyle.

Dans la formule générale (I), $R_1$ et $R_2$ peuvent représenter également un radical carbocyclique monocyclique saturé ou comprenant 1 ou 2 insaturations dans le cycle, ayant généralement de 3 à 7 atomes de carbone, de préférence, 5 ou 6 atomes de carbone dans le cycle.

Comme exemples préférés de radicaux R, on peut citer les radicaux cyclopentyle ou cyclopentène-yle, cyclohexyle ou cyclohexène-yle.

Dans le cas où $R_1$ et $R_2$ représentent un radical carbocyclique, monocyclique, saturé ou insaturé, il est possible que l'un ou plusieurs des atomes du carbone du cycle soient remplacés par un hétéroatome, de préférence, oxygène, azote ou soufre ou par un groupe fonctionnel, de préférence carbonyle ou ester, conduisant ainsi à un composé hétérocyclique, monocyclique. Le nombre d'atomes dans le cycle peut varier largement de 3 à 8 atomes mais il est de préférence égal à 5 ou 6 atomes.

Les radicaux $R_1$ et $R_2$ peuvent être également carbocyclique, polycyclique, de préférence bicyclique ce qui signifie qu'au moins deux cycles ont deux atomes de carbone en commun. Dans le cas des radicaux polycycliques, le nombre d'atomes de carbone dans chaque cycle varie entre 3 et 6 : le nombre total d'atomes de carbone étant égal de préférence à 7.

On donne ci-après des exemples de structure bicyclique, couramment rencontrée :

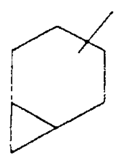
[4,1,0]

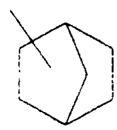
[2,2,1]

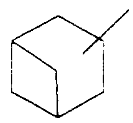
[3,1,1]

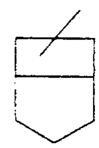
[3,2,0]

Les radicaux $R_1$ et $R_2$ peuvent être également hétérocyclique, polycyclique, de préférence bicyclique ce qui signifie qu'au moins deux cycles ont deux atomes en commun. Dans ce cas, le nombre d'atomes dans chaque cycle varie entre 3 et 6 et est plus préférentiellement égal à 5 ou 6.

Les radicaux $R_1$ et $R_2$ peuvent représenter préférentiellement un radical carbocyclique aromatique, et notamment benzénique répondant à la formule générale :

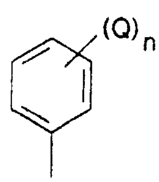

dans ladite formule :

- n est un nombre entier de 0 à 5, de préférence de 0 à 3,
- Q représente R, l'un des groupes ou fonctions suivantes :
  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical de formule :

$$-R_3-OH$$

$$-R_3-O-R_4$$

$$-R_3-CO-R_4$$

$-R_3-COOR_4$

$-R_3-CHO$

$-R_3-NO_2$

$-R_3-CN$

$-R_3-N(R_4)_2$

$-R_3-CO-N(R_4)_2$

$-R_3-PO-(OR_4)_2$

$-R_3-SH$

$-R_3-X$

$-R_3-CF_3$

dans lesdites formules, $R_3$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; les radicaux $R_4$, identiques ou différents, représentent un atome d'hydrogène ou un radical alkyle linéaire ou ramifié ayant de 1 à 6 atomes de carbone, un radical benzyle ou un radical phényle ; X symbolise un atome d'halogène, de préférence un atome de chlore, de brome ou de fluor,

- Q représente R', l'un des radicaux plus complexes suivants :

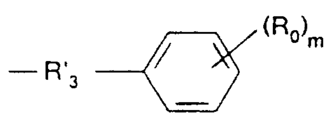

dans lequel :

- . m est un nombre entier de 0 à 5, de préférence de 0 à 3,
- . $R_0$ ayant la signification donnée précédemment pour R,
- . $R'_3$ représente un lien valentiel ; un groupe hydrocarboné divalent, linéaire ou ramifié, saturé ou insaturé ayant de 1 à 6 atomes de carbone tel que par exemple, méthylène, éthylène, propylène, isopropylène, isopropylidène ou l'un des groupes suivants :

$-O-; -CO- ; COO- ; -NR_4- ; -CO-NR_4- ; -S- ; -SO_2- ; -NR_4-CO-;$

dans lesdites formules $R_4$ ayant la signification donnée précédemment.

Comme exemples de radicaux $R_1$ et $R_2$ répondant à la formule (II), on peut mentionner plus précisément les radicaux phényle, tolyle ou xylyle, 1-méthoxyphényle, 2-nitrophényle et les radicaux biphényle, 1,1'-méthylènebiphényle, 1,1'-isopropylidènebiphényle, 1,1'-carboxybiphényle, 1,1'-oxybiphényle, 1,1'-iminobiphényle: lesdits radicaux pouvant être substitués par un ou plusieurs radicaux R tels que prédéfinis.

$R_1$ et $R_2$ peuvent également représenter un radical hydrocarboné aromatique polycyclique ; les cycles pouvant former entre eux des systèmes ortho-condensés, ortho- et péri-condensés. On peut citer plus particulièrement, un radical naphtalénique.

Dans la formule générale (I), $R_1$ et $R_2$ peuvent également représenter un radical hétérocyclique, saturé, insaturé ou aromatique, comportant notamment 5 ou 6 atomes dans le cycle dont 1 ou 2 hétéroatomes tels que les atomes d'azote, de soufre et d'oxygène.

$R_1$ et $R_2$ peuvent aussi représenter un radical hétérocyclique aromatique polycyclique défini comme étant soit un radical constitué par au moins 2 hétérocycles aromatiques ou non contenant au moins un hétéroatome dans chaque cycle et formant entre eux des systèmes ortho- ou ortho- et péricondensés ou soit un radical constitué par au moins un cycle hydrocarboné aromatique ou non et au moins un hétérocycle aromatique ou non formant entre eux des systèmes ortho- ou ortho- et péricondensés.

Il est à noter que si les radicaux $R_1$ et $R_2$ comprennent un cycle quelconque, il est possible que ce cycle porte un substituant. La nature du substituant est quelconque dans la mesure où il n'interfère pas au niveau du produit désiré. R illustre le type de substituants couramment rencontrés. Les substituants portés le plus souvent par le cycle sont un ou plusieurs radicaux alkyle ou alkoxy ayant de préférence de 1 à 4 atomes de carbone, un atome d'halogène.

Parmi les composés de formule (V), ceux qui sont préférés ont un radical phényle comme radicaux $R_1$ ou $R_2$

Dans la formule (V), il est possible que $R_1$ et $R_2$ forment ensemble et avec l'atome de carbone qui porte le groupe fonctionnel, un système cyclique, monocyclique ou polycyclique.

Ainsi, le composé de formule (V) peut être :

- un composé monocyclique, carbocyclique, saturé ou insaturé,
- un composé polycyclique comprenant au moins deux carbocycles, saturés et/ou insaturés,
- un composé comprenant au moins deux cycles saturés et/ou insaturés : un ou plusieurs des atomes de carbone pouvant être remplacés par un hétéroatome,
- un composé polycyclique comprenant au moins deux carbocycles dont l'un d'eux est aromatique.

Lorsqu'il s'agit d'un composé monocyclique, le nombre d'atomes de carbone dans le cycle peut varier largement de 3 à 20 atomes de carbone mais il est de préférence de 5 ou 6 atomes de carbone.

Le carbocycle peut être saturé ou comprenant 1 ou 2 insaturations dans le cycle, de préférence de 1 à 2 doubles liaisons qui sont le plus souvent en position $\alpha$ du groupe fonctionnel.

Le composé peut être également polycyclique, de préférence bicyclique ce qui signifie qu'au moins deux cycles ont deux atomes de carbone en commun.

Dans le cas des composés polycycliques, la condensation en carbone de chaque cycle est plus faible, généralement de 3 à 8 mais est égale, de préférence à 5 ou 6 atomes de carbone.

Le composé polycyclique peut comprendre au moins deux cycles saturés et/ou insaturés : un ou plusieurs (de préférence deux) des atomes de carbone pouvant être remplacés par un hétéroatome, de préférence un atome d'oxygène ou d'azote.

Le composé polycyclique peut comprendre au moins deux carbocycles dont l'un d'eux est aromatique, le cycle aromatique étant de préférence un cycle benzénique.

Le ou les cycles peuvent être porteurs d'un ou plusieurs substituants.

Le nombre de substituants présents sur le cycle dépend de la condensation en carbone du cycle et de la présence ou non d'insaturations sur le cycle.

Le nombre maximum de substituants susceptibles d'être portés par un cycle, est aisément déterminé par l'Homme du Métier.

En ce qui concerne la nature des substituants, des exemples de substituants sont donnés ci-dessus pour R mais cette liste ne présente pas de caractère limitatif.

Plus précisément, lorsque le composé de formule (V) est un composé carbocyclique monocyclique saturé, le nombre d'atomes de carbone dans le cycle peut varier largement de 3 à 20 atomes de carbone mais il est de préférence de 5 ou 6 atomes de carbone. Il peut y avoir présence d'un ou de deux groupes fonctionnels (C=Z) sur le cycle. Le groupe fonctionnel est de préférence porté par un carbocycle saturé ayant de 5 ou 6 atomes de carbone.

Le carbocycle saturé peut porter des substituants. Le nombre de substituants sur chaque cycle, peut varier largement de 1 à 5. Il est généralement de 1 ou 2.

Le carbocycle peut être insaturé et comprendre 1 ou 2 insaturations dans le cycle, de préférence de 1 à 2 doubles liaisons qui sont le plus souvent en position $\alpha$ du groupe fonctionnel. Il comprend de 4 à 20 atomes de carbone. Il peut y avoir présence d'un ou de deux groupes fonctionnels sur le cycle. Le groupe fonctionnel est de préférence porté par un carbocycle insaturé ayant de 5 ou 6 atomes de carbone.

Le carbocycle insaturé peut porter des substituants. Le nombre de substituants sur chaque cycle, peut varier largement de 1 à 5. Il est généralement de 1 ou 2.

Le composé peut être également polycyclique, de préférence bicyclique.

Le composé polycyclique peut comprendre au moins deux cycles saturés et/ou insaturés : un ou plusieurs (de préférence deux) des atomes de carbone pouvant être remplacés par un hétéroatome, de préférence un atome d'oxygène ou d'azote.

Le composé carbocyclique polycyclique, de préférence, bicyclique peut comprendre deux carbocycles saturés, ayant chacun de préférence de 4 à 8 atomes de carbone. Il peut y avoir présence d'un groupe fonctionnel sur l'un ou les deux cycles. Il est également possible qu'il y ait deux groupes fonctionnels sur le même cycle. Le groupe fonctionnel est de préférence porté par un ou deux carbocycles saturés ayant de 5 ou 6 atomes de carbone.

Dans ces composés polycycliques, un ou plusieurs atomes de carbone, (de préférence deux), peuvent être remplacés par un hétéroatome, de préférence un atome d'azote ou d'oxygène.

Le ou les cycles de ces composés polycycliques peuvent porter des substituants. Le nombre de substituants sur chaque cycle est généralement de 1 à 4, de préférence, de 1 ou 2.

Le composé cyclique peut être un composé carbocyclique bicyclique comprenant deux carbocycles, ayant chacun de préférence de 4 à 7 atomes de carbone, l'un saturé, l'autre insaturé, généralement avec une seule double liaison. Le groupe fonctionnel peut intervenir aussi bien dans le cycle saturé qu'insaturé ou sur les deux. Le groupe fonctionnel est de préférence porté par un carbocycle saturé ou insaturé, ayant de 5 ou 6 atomes de carbone.

Le ou les cycles de ces composés polycycliques peuvent porter des substituants. Le nombre de substituants sur chaque cycle est généralement de 1 à 3, de préférence, de 1 ou 2.

Le composé carbocyclique polycyclique, de préférence, bicyclique peut comprendre deux carbocycles insaturés, ayant chacun de préférence de 5 ou 6 atomes de carbone. Il peut y avoir présence d'un groupe fonctionnel sur l'un des deux cycles.

Dans ces composés polycycliques, un ou plusieurs atomes de carbone, (de préférence deux), peuvent être remplacés par un hétéroatome, de préférence un atome d'azote ou d'oxygène.

Le ou les cycles de ces composés polycycliques peuvent porter des substituants. Le nombre de substituants sur chaque cycle est généralement de 1 à 4, de préférence, de 1 ou 2.

Le composé carbocyclique polycyclique peut comprendre au moins un carbocycle aromatique, de préférence, un cycle benzénique et un carbocycle ayant de préférence de 4 à 7 atomes de carbone et comprenant un ou deux groupes fonctionnels.

Le composé polycyclique est de préférence un composé bicyclique comprenant un cycle benzénique et un carbocycle de 5 ou 6 atomes de carbone comprenant un ou deux groupes fonctionnels.

Les deux cycles de ce radical bicyclique peuvent porter des substituants. Le nombre de substituants sur chaque cycle est généralement de 1 à 4, de préférence, de 1 ou 2.

Plus précisément, le composé de formule (V) correspond à un composé cétonique répondant à la formule (Va) :

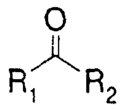 (Va)

dans ladite formule (Va) :

- $R_1$ est différent de $R_2$, les radicaux $R_1$ et $R_2$ représentent un radical hydrocarboné ayant de 1 à 30 atomes de carbone comprenant éventuellement une autre fonction cétone et/ou acide, ester, thioacide, thioester.
- $R_1$ et $R_2$ peuvent former un cycle carbocyclique ou hétérocyclique, substitué ou non, ayant de 5 à 6 atomes.

Une première classe de substrats auxquels s'appliquent plus préférentiellement le procédé de l'invention sont les cétones éventuellement fonctionnalisées.

Par ce dernier terme, on entend la présence d'un quelconque groupe fonctionnel en position α, β, γ ou δ, à l'exception de tout groupe comprenant un groupe carbonyle.

Lesdites cétones peuvent être symbolisées par la formule chimique suivante:

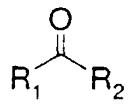
(Va$_1$)

dans ladite formule (Va$_1$):

- $R_1$ et $R_2$ représentent :
  - un radical alkyle, linéaire ou ramifié, ayant de 1 à 12 atomes de carbone, de préférence de 1 à 4 atomes de carbone, tel que méthyle, éthyle, propyle, isopropyle, butyle, isobutyle, sec-butyle, tert-butyle,
  - un radical alcényle ayant de 2 à 12 atomes de carbone, de préférence, un radical allyle,
  - un radical phényle, naphtyle ou benzyle éventuellement substitué,
  - un radical triphénylméthyle,
  - un radical de formule :

$-R_3-OH$

$-R_3-O-R_4$

$-R_3-CO-R_4$

$-R_3-COOR_4$

$-R_3-CHO$

$-R_3-NO_2$

$-R_3-CN$

$-R_3-N(R_4)_2$

$-R_3-CO-N(R_4)_2$

$-R_3-PO-(OR_4)_2$

$-R_3-SH$

$-R_3-X$

$-R_3-CF_3$

dans lesdites formules, $R_3$ représente un lien valentiel ou un radical hydrocarboné divalent, linéaire ou ramifié, saturé ou insaturé, ayant de 1 à 6 atomes de carbone tel que, par exemple, méthylène, éthylène, propylène,

isopropylène, isopropylidène ; les radicaux $R_4$, identiques ou différents, représentent un atome d'hydrogène ou un radical alkyle linéaire ou ramifié ayant de 1 à 6 atomes de carbone, un radical benzyle ou un radical phényle ; X symbolise un atome d'halogène, de préférence un atome de chlore, de brome ou de fluor,

- $R_1$ et $R_2$ peuvent former un cycle carbocyclique ou hétérocyclique, substitué ou non, ayant de 5 à 6 atomes.

Si le ou les radicaux $R_1$ et $R_2$ présentent une chaîne hydrocarbonée, celle-ci peut être éventuellement interrompue par un hétéroatome (par exemple, l'oxygène ou l'azote), par un groupe fonctionnel et/ou porteuse d'un substituant (par exemple, un halogène, un groupe trifluorométhyle ou une fonction ester).

Dans le cas où le ou les radicaux $R_1$ et $R_2$ présentent un cycle tel que benzénique ou autre, il est possible qu'il y est des substituants sur ce cycle. N'importe quel substituant peut être présent et l'on peut se référer aux significations de R. Il s'agit le plus souvent d'un radical alkyle inférieur, un radical alkoxy inférieur, un groupe hydroxyle et/ou un atome d'halogène, notamment de fluor ou de chlore.

Comme exemples de cétones répondant à la formule ($Va_1$), on peut mentionner entre autres :

- méthylphénylcétone,
- isopropylphénylcétone,
- cyclopropylphénylcétone,
- allylphénylcétone,
- p-méthylphénylméthylcétone,
- benzylphénylcétone,
- phényltriphénylméthylcétone,
- o-bromoacétophénone,
- α-bromoacétone,
- α-dibromoacétone,
- α-chloroacétone,
- α-dichloroacétone,
- α-trichloroacétone,
- 1-chloro-3,3-dichloroacétone,
- 1-chloro-2-oxobutane,
- 1-fluoro-2-oxobutane,
- 1-chloro-3-méthyl-2-butanone,
- α-chloroacétophénone,
- 1-chloro-3-phénylacétone,
- α-méthylaminoacétone,
- α-diméthylaminoacétone,
- 1-butylamino-2-oxopropane,
- 1-dibutylamino-2-oxopropane.
- 1-méthylamino-2-oxobutane,
- 1-diméthylamino-2-oxobutane,
- 1-diméthylamino-3-méthyl-2-oxobutane,
- 1-diméthylamino-2-oxopentane,
- α-diméthylaminoacétophénone,
- α-hydroxyacétone,
- 1-hydroxy-3-méthyl-2-butanone,
- 1-hydroxy-2-oxobutane,
- 1-hydroxy-2-oxopentane,
- 1-hydroxy-2-oxohexane,
- 1-hydroxy-2-oxo-3-méthylbutane,
- α-hydroxyacétophénone,
- 1-hydroxy-3-phénylacétone,
- α-méthoxyacétone,
- α-méthoxyacétophénone,
- α-méthoxyacétophénone,
- α-éthoxyacétone,
- α-butoxyacétophénone,
- α-chloro-p-méthoxyacétophénone,
- α-naphténone,
- 1-éthoxy-2-oxobutane,

- 1-butoxy-2-oxobutane,
- α-diméthoxyphosphorylacétone,
- 3-oxotétrahydrothiophène.

L'invention s'applique également, tout à fait bien pour effectuer l'hydrogénation des composés dicétoniques présentant un groupe carbonyle en position α, β, γ ou δ, par rapport à un premier groupe carbonyle. Les composés dicétoniques répondent plus particulièrement à la formule ($Va_2$):

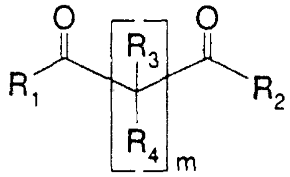

($Va_2$)

dans ladite formule ($Va_2$):

- m est égal à 0,1,2 ou 3, de préférence, égal à 0 ou 1,
- les radicaux $R_1$ et $R_2$ différents, représentent:
  - . un radical alkyle, linéaire ou ramifié, ayant de 1 à 12 atomes de carbone, éventuellement porteur d'un atome d'halogène, de préférence de 1 à 4 atomes de carbone, tel que méthyle, éthyle, propyle, isopropyle, butyle, isobutyle, sec-butyle, tert-butyle,
  - . un radical alcényle ayant de 2 à 12 atomes de carbone, de préférence, un radical allyle,
  - . un radical phényle, naphtyle ou benzyle éventuellement substitué,
  - . un radical de formule :

$$-R_5-OH$$

$$-R_5-O-R_6$$

$$-R_5-CO-R_6$$

$$-R_5-COOR_6$$

$$-R_5-N(R_6)_2$$

$$-R_5-CO-N(R_6)_2$$

$$-R_5-PO-(OR_6)_2$$

$$-R_5-SH$$

$$-R_5-X$$

$$-R_5-CF_3$$

dans lesdites formules, $R_5$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; les radicaux $R_6$, identiques ou différents, représentent un atome d'hydrogène ou un radical alkyle linéaire ou ramifié ayant de 1 à 6 atomes de carbone, un radical benzyle ou un radical phényle ; X symbolise un atome d'halogène, de préférence un atome de chlore, de brome ou de fluor,

. l'un des radicaux $R_1$ et $R_2$ pouvant représenter un atome d'hydrogène,

- les radicaux $R_3$ et $R_4$, identiques ou différents, représentent :

. un atome d'hydrogène,
. un radical alkyle, linéaire ou ramifié, ayant de 1 à 12 atomes de carbone, éventuellement porteur d'un atome d'halogène, de préférence de 1 à 4 atomes de carbone, tel que méthyle, éthyle, propyle, isopropyle, butyle, isobutyle, sec-butyle, tert-butyle,
. un atome d'halogène,
. un groupe du type $-R_5-COOR_6$ dans lequel $R_5$ et $R_6$ ayant la signification donnée précédemment,

- $R_1$ ou $R_2$ et $R_3$ ou $R_4$ peuvent former un cycle carbocyclique ou hétérocyclique, substitué ou non, ayant de 5 à 6 atomes.

Si le ou les radicaux $R_1$ et $R_2$ présentent une chaîne hydrocarbonée, celle-ci peut être éventuellement interrompue par un hétéroatome (par exemple, l'oxygène ou l'azote), par un groupe fonctionnel et/ou porteuse d'un substituant (par exemple, un halogène, un groupe trifluorométhyle ou une fonction ester).
Dans le cas où le ou les radicaux $R_1$ et $R_2$ présentent un cycle, ils peuvent être substitués comme décrit pour la formule ($Va_1$).
Comme exemples de composés dicétoniques de formule ($Va_2$), on peut citer notamment :

- α-formylacétone,
- diacétyle,
- 3,4-dioxohexane,
- 4,5-dioxooctane,
- 1-phényl-1,2-dioxopropane,
- 1-phényl-2,3-dioxobutane,
- dibenzoyle,
- p-méthoxydibenzoyle
- 1,2-cyclopentanedione,
- 1,2-cyclohexanedione,
- acétylacétone,
- 3,5-heptanedione,
- 4,6-nonanedione,
- 5,7-undecadione,
- 2,4-hexanedione,
- 2,4-heptanedione,
- 2,4-octanedione,
- 2,4-nonanedione,
- 3,5-nonanedione,
- 3,5-décanedione,
- 2,4-dodécanedione,
- 1-phényl-1,3-butanedione,
- 1-phényl-1,3-pentanedione,
- 1-phényl-1,3-hexanedione,
- 1-phényl-1,3-heptanedione,
- 3-méthyl-2,4-pentanedione,
- 1,3-diphényl-1,3-propanedione,
- 1,5-diphényl-2,4-pentanedione,
- 1,3-di(trifluorométhyl)-1,3-propanedione,
- 3-chloro-2,4-pentanedione
- 1,5-dichloro-2,4-pentanedione,
- 1,5-dihydroxy-2,4-pentanedione,

- 1,5-dibenzyloxy-2,4-pentanedione,
- 1,5-diamino-2,4-pentanedione,
- 1,5-di(méthylamino)2,4-pentanedione,
- 1,5-di(diméthylamino)-2,4-pentanedione,
- 3,5-dioxo-hexanoate de méthyle.
- 3-carbométhoxy-2,4-pentanedione,
- 3-carboéthoxy-2,4-pentanedione,
- 1,3-cyclopentanedione,
- 1,3-cyclohexanedione,
- 1,3-cycloheptanedione,
- 5-carboéthoxy-1,3-cyclopentanedione,
- 2-acétyl-1-cyclopentanone,
- 2-acétyl-1-cyclohexanone.

L'invention s'applique également, tout à fait bien pour effectuer l'hydrogénation des cétoacides ou dérivés et des cétothioacides ou dérivés avec un groupe fonctionnel (acide, ester, thioacide ou thioester) en position $\alpha$, $\beta$, $\gamma$ ou $\delta$ par rapport au groupe carbonyle. L'invention convient tout à fait bien pour effectuer l'hydrogénation des composés répondant à la formule ($Va_3$) ou ($Va_4$) :

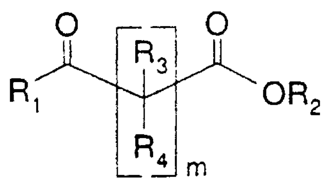

($Va_3$) ou

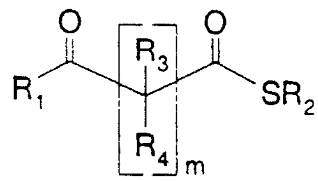

($Va_4$)

dans lesdites formules ($Va_3$) ou ($Va_4$):

- m est égal à 0,1,2 ou 3, de préférence, égal à 0 ou 1,
- le radical $R_1$ représente:
  . un radical alkyle, linéaire ou ramifié, ayant de 1 à 12 atomes de carbone, éventuellement porteur d'un atome d'halogène, de préférence de 1 à 4 atomes de carbone, tel que méthyle, éthyle, propyle, isopropyle, butyle, isobutyle, sec-butyle, tert-butyle,
  . un radical alcényle ayant de 2 à 12 atomes de carbone, de préférence, un radical allyle,
  . un radical phényle, naphtyle ou benzyle éventuellement substitué,
  . un radical de formule :

$$-R_5\text{-}OH$$

$$-R_5\text{-}O\text{-}R_6$$

$$-R_5\text{-}CO\text{-}R_6$$

$$-R_5\text{-}COOR_6$$

$$-R_5\text{-}N(R_6)_2$$

$$-R_5\text{-}CO\text{-}N(R_6)_2$$

$-R_5-PO-(OR_6)_2$

$-R_5-SH$

$-R_5-X$

$-R_5-CF_3$

dans lesdites formules, $R_5$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; les radicaux $R_6$, identiques ou différents, représentent un atome d'hydrogène ou un radical alkyle linéaire ou ramifié ayant de 1 à 6 atomes de carbone, un radical benzyle ou un radical phényle ; X symbolise un atome d'halogène, de préférence un atome de chlore, de brome ou de fluor,

- le radical $R_2$ représente :
  - . un atome d'hydrogène,
  - . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  - . un radical benzyle.
  - . un radical phényle,

- les radicaux $R_3$ et $R_4$, identiques ou différents, représentent :
  - . un atome d'hydrogène,
  - . un radical alkyle, linéaire ou ramifié, ayant de 1 à 12 atomes de carbone, de préférence de 1 à 4 atomes de carbone, tel que méthyle, éthyle, propyle, isopropyle, butyle, isobutyle, sec-butyle, tert-butyle,
  - . un atome d'halogène,
  - . un groupe du type $-R_5-COOR_6$ dans lequel $R_5$ et $R_6$ ayant la signification donnée précédemment,
  - . un groupe du type $-R_5-CO-N(R_6)_2$ dans lequel $R_5$ et $R_6$ ayant la signification donnée précédemment,

- $R_1$ et $R_2$ ou $R_1$ et $R_3$ ou $R_1$ et $R_4$ ou $R_2$ et $R_4$ peuvent former un cycle carbocyclique ou hétérocyclique, substitué ou non, ayant de 5 à 6 atomes.

Si le ou les radicaux $R_1$ et $R_2$ présentent une chaîne hydrocarbonée, celle-ci peut être éventuellement interrompue par un hétéroatome (par exemple, l'oxygène ou l'azote), par un groupe fonctionnel et/ou porteuse d'un substituant (par exemple, un halogène, un groupe trifluorométhyle ou une fonction ester).

Dans le cas où le ou les radicaux $R_1$ et $R_2$ présentent un cycle, ils peuvent être substitués comme décrit pour la formule ($Va_1$).

Comme exemples plus particuliers de composés répondant aux formules ($Va_3$) ou ($Va_4$), on peut citer par exemple :

- acide 2-acétylbenzoïque,
- acide pyruvique,
- acide 2-oxobutanoïque,
- acide 3-méthyl-2-oxobutanoïque,
- acide phénylglyoxylique,
- acide phénylpyruvique,
- acide p-méthoxyphénylpyruvique,
- acide 3,4-diméthoxyphénylpyruvique,
- acétoacétate de méthyle,
- acétoacétate d'éthyle,
- acétoacétate de n-propyle,
- acétoacétate d'isopropyle,

- acétoacétate de n-butyle,
- acétoacétate de t-butyle,
- acétoacétate de n-pentyle,
- acétoacétate de n-hexyle,
- acétoacétate de n-heptyle,
- acétoacétate de n-octyle,
- 3-oxopentanoate de méthyle,
- 3-oxohexanoate de méthyle,
- 3-oxoheptanoate de méthyle,
- 3-oxooctanoate d'éthyle,
- 3-oxononanoate d'éthyle,
- 3-oxodécanoate d'éthyle,
- 3-oxoundécanoate d'éthyle,
- 3-oxo-3-phénylpropanoate d'éthyle,
- 4-phényl-3-oxobutanoate d'éthyle,
- 5-phényl-3-oxopentanoate de méthyle,
- 3-oxo-3-p-méthoxyphénylpropanoate d'éthyle,
- 4-chloroacétoacétate de méthyle,
- 4-chloroacétoacétate d'éthyle,
- 4-fluoroacétoacétate de méthyle,
- 3-trifluorométhyl-3-oxopropanoate d'éthyle,
- 4-hydroxy-3-oxobutanoate d'éthyle,
- 4-méthoxyacétoacétate de méthyle,
- 4-tert-butoxyacétoacétate de méthyle,
- 4-benzyloxy-3-oxobutanoate de méthyle,
- 4-benzyloxy-3-oxobutanoate d'éthyle,
- 4-amino-3-oxobutanoate de méthyle,
- 3-méthylamino-3-oxobutanoate d'éthyle,
- 4-diméthylamino-3-oxobutanoate de méthyle,
- 4-diméthylamino-3-oxobutanoate d'éthyle,
- 2-méthylacétoacétate de méthyle,
- 2-méthylacétoacétate d'éthyle,
- 2-chloroacétoacétate d'éthyle,
- 2-acétylsuccinate de diéthyle,
- 2-acétylglutarte de diéthyle,
- acétylmalonate de diméthyle,
- acétoacétate de thiométhyle,
- acétoacétate de thioéthyle,
- acétoacétate de thiophényle,
- pyruvate de méthyle,
- 3-méthyl-2-oxobutanoate d'éthyle,
- phénylglyoxolate d'éthyle,
- phénylpyruvate de méthyle,
- phénylpyruvate d'éthyle,
- 3-oxobutanoic diméthylamide,
- 3-oxobutanoic benzylamide,
- 2-carboéthoxy-cyclopentanone,
- 2-carboéthoxy-cyclohexanone,
- cétopentalactone.
- acide 4-oxopentanoïque,
- acide 4-oxohexanoique,
- acide 4-oxoheptanoique,
- acide 4-oxodécanoîque,
- acide 4-oxododécanoïque,
- acide 4-phényl-4-oxybutyrique,
- acide 4-p-méthoxyphényl-4-oxybutyrique,
- acide 4-(3,4-diméthoxyphényl)-4-oxobutyrique,
- acide 4-(3,4,5-triméthoxyphényl)-4-oxobutyrique,

- acide 4-p-chlorophényl-4-oxybutyrique,
- acide 4-phényl-4-oxobutyrique.

Il est à noter que lorsque l'on a à faire l'hydrogénation asymétrique d'un γ-cétoacide ou dérivé, le produit obtenu est généralement un dérivé de γ-butyrolactone et dans le cas d'un δ-cétoacide, il s'agit d'un dérivé de valérolactone.

Comme autres exemples de cétones, on peut mentionner entre autres, les composés cétoniques cycliques, saturés ou insaturés, monocycliques ou polycycliques suivants :

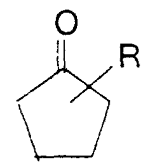
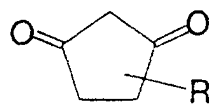
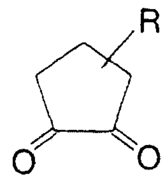

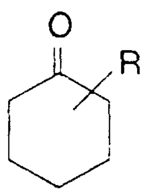
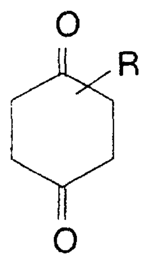
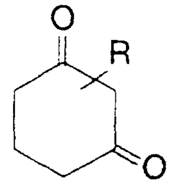
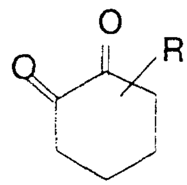

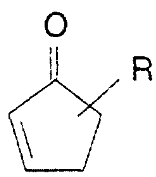
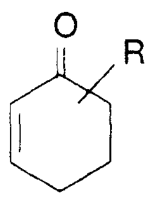
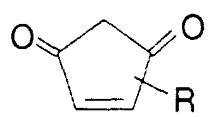
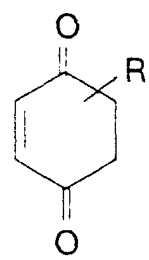

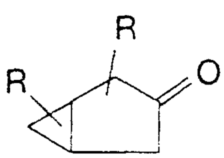
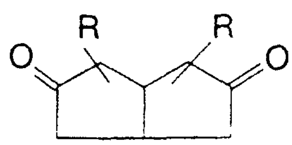
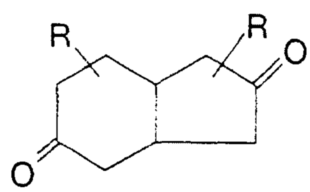

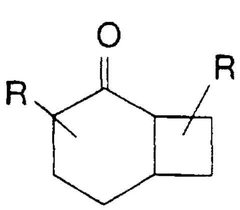
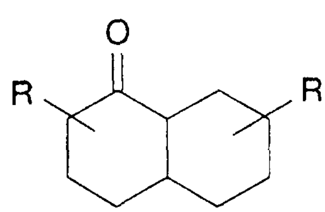
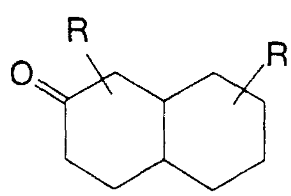

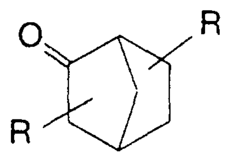
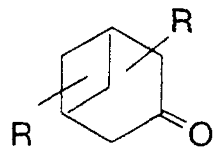
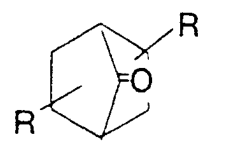
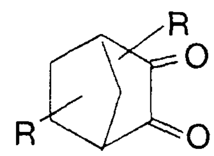

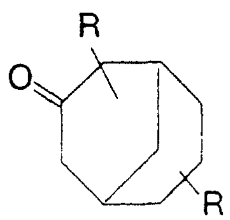
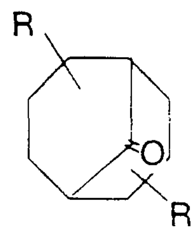
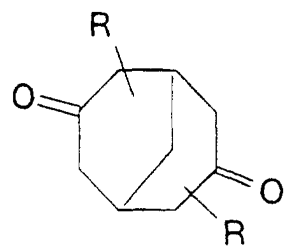

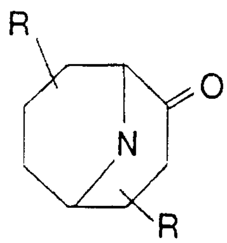
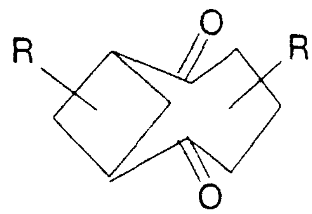

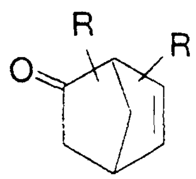
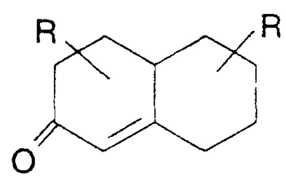
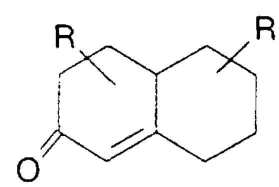

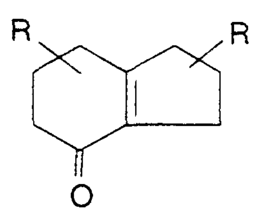
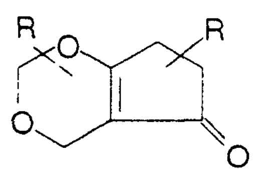
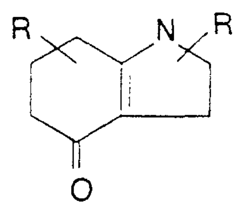
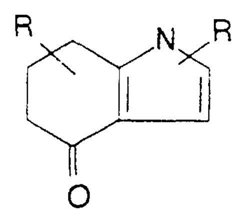

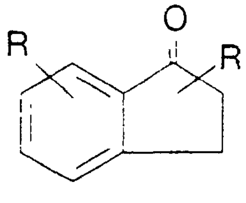
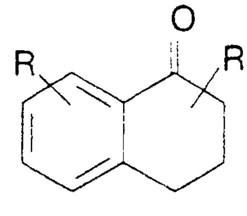
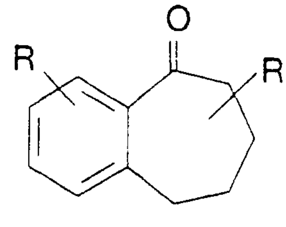

On peut également mettre en oeuvre des cétones de type stéroïde (par exemple 3-cholestanone, 5-cholesten-3-one).

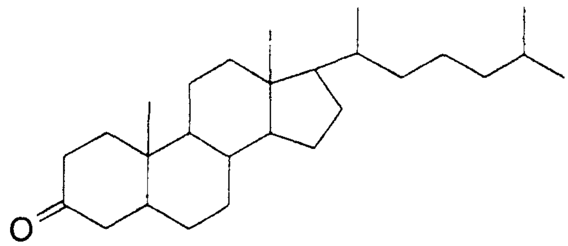

Une autre classe de substrats auxquels s'applique le procédé de l'invention sont les composés de formule (V) dans laquelle Z symbolise un atome d'azote ou un groupe fonctionnel comprenant un atome d'azote et qui sont représentés par la formule (Vb) :

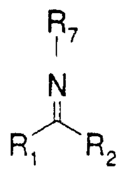
(Vb)

dans ladite formule (Vb) :

- $R_1$ différent de $R_2$, ont la signification donnée précédemment,
- $R_7$ représente :
    - . un atome d'hydrogène,
    - . un groupe hydroxyle,
    - . un groupe $OR_8$
    - . un radical hydrocarboné $R_8$,
    - . un groupe de formule

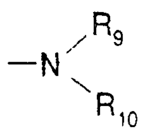

    - . un groupe de formule

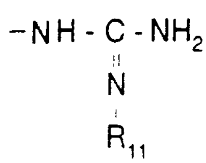

avec $R_8$, $R_9$, $R_{10}$, et $R_{11}$ qui représentent un atome d'hydrogène ou un groupement hydrocarboné ayant de 1 à 30 atomes de carbone.

Il est à noter que l'invention inclut également des substrats qui comprennent plusieurs groupes fonctionnels définis ci-dessus et qui peuvent être dérivés de composés dicétoniques, les groupes fonctionnels étant en position $\alpha$, $\beta$, $\gamma$ ou $\delta$.

Les composés mis en oeuvre préférentiellement dans le procédé de l'invention répondent plus particulièrement aux formules suivantes :

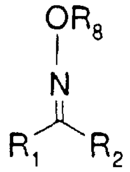 (Vb$_1$)

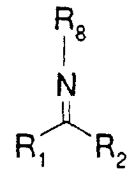 (Vb$_2$)

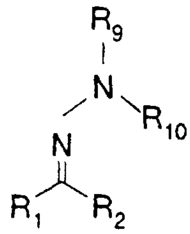 (Vb$_3$)

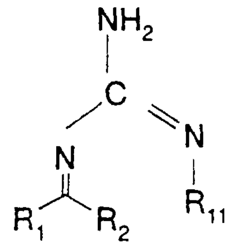 (Vb$_4$)

dans lesdites formules (Vb$_1$) à (Vb$_4$):

- les radicaux $R_1$ et $R_2$, $R_8$ à $R_{11}$ représentent:
  - . un radical alkyle linéaire ou ramifié ayant de 1 à 12 atomes de carbone,
  - . un radical cycloalkyle ayant de 5 à 12 atomes de carbone,
  - . un radical aryle ayant de 6 à 12 atomes de carbone,
  - . un radical aralkyle ayant de 7 à 12 atomes de carbone,
  - . un radical aryle ayant de 6 à 12 atomes de carbone porteurs de substituants tels qu'un radical alkyle ou alkoxy ayant de 1 à 4 atomes de carbone, un groupe amino, ($C_1$-$C_4$)alkylamino ou di-($C_1$-$C_4$)alkylamino, un groupe nitro, un atome d'halogène, un groupe ($C_1$-$C_4$)alkoxycarbonyle,
  - . un radical aryle ayant de 6 à 12 atomes de carbone,
  - . un radical hétérocyclique saturé ou insaturé,
  - . un radical alcanoyle ayant de 1 à 12 atomes de carbone,
  - . un radical arylcarbonyle ayant de 6 à 12 atomes de carbone,
  - . un radical arylalcanoyle ayant de 6 à 12 atomes de carbone,

- $R_1$ et $R_2$, $R_1$ et $R_8$, $R_2$ et $R_8$, $R_1$ et $R_9$, $R_2$ et $R_{10}$, $R_1$ et $R_{11}$, $R_2$ et $R_{11}$ peuvent former un cycle carbocyclique ou hétérocyclique, substitué ou non, monocyclique ou polycyclique, ayant de 5 à 6 atomes dans chaque cycle.

Dans les formules (Vb$_1$) à (Vb$_4$), les radicaux $R_1$ et $R_2$, $R_8$ à $R_{11}$, identiques ou différents représentent préférentiellement :

- . un radical alkyle linéaire ou ramifié ayant de 1 à 4 atomes de carbone,
- . un radical cyclopentyle ou cyclohexyle,
- . un radical phényle
- . un radical benzyle ou phényléthyle,
- . un radical phényle porteur de substituants tels qu'un radical alkyle ou alkoxy ayant de 1 à 4 atomes de carbone, un groupe amino, ($C_1$-$C_4$)alkylamino ou di-($C_1$-$C_4$)alkylamino, un groupe nitro, un atome d'halogène, un groupe ($C_1$-$C_4$)alkoxycarbonyle,
- . un radical naphtyle,
- . un radical hétérocyclique oxygéné ou azoté saturé ou insaturé à 5 ou 6 atomes,
- . un radical acétyle, un radical benzoyle,
- . un radical arylalcanoyle ayant de 6 à 12 atomes de carbone,

Les composés répondant à la formule (Vb$_1$) sont de type oxime. Comme exemples. on peut citer entre autres, l'oxime de l'acétophénone :

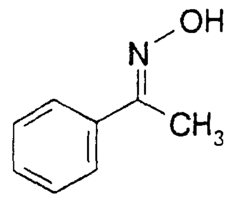

Les composés répondant à la formule ($Vb_2$) sont des imines. Comme exemples plus spécifiques, on peut mentionner :

. comme N-alkylcétoimine

- N-isobutyl-2-iminopropane
- N-isobutyl-1-méthoxy-2-iminopropane

. comme N-arylalkylcétoimine

- N-benzyl-1-imino-1-(phényl)éthane
- N-benzyl-1-imino-1-(4-méthoxyphényl)éthane
- N-benzyl-1-imino-1-(2-méthoxyphényl)éthane

. comme N-arylcétoimine

- N-phényl-2-iminopentane
- N-(2,6-diméthylphényl)-2-iminopentane
- N-(2,4,6-triméthylphényl)-2-iminopentane
- N-phényl-1-imino-1-phényléthane
- N-phényl-1-méthoxy-2-iminopropane
- N-(2,6-diméthylphényl)-1-méthoxy-2-iminopropane
- N-(2-méthyl-6-éthylphényl)-1-méthoxy-2-iminopropane

En ce qui concerne les composés de formule ($Vb_3$), il s'agit de composés de type hydrazone, éventuellement N-acylés ou N-benzoylés et l'on peut citer plus particulièrement :

- 1-cyclohexyl-1-(2-benzoylhydrazono)éthane,
- 1-phényl-1-(2-benzoylhydrazono)éthane,
- 1-p-méthoxyphényl-1-(2-benzoylhydrazono)éthane,
- 1-p-éthoxyphényl-1-(2-benzoylhydrazono)éthane,
- 1-p-nitrophényl-1-(2-benzoylhydrazono)éthane,
- 1-p-bromophényl-1-(2-benzoylhydrazono)éthane,
- 1-p-carboéthoxyphényl-1-(2-benzoylhydrazono)éthane,
- 1,2-diphényl-1-(2-benzoylhydrazono)éthane,
- 1,2-diphényl-1-(2-benzoylhydrazono)éthane,
- 3-méthyl-2-(2-p-diméthylaminobenzoylhydrazono)butane,
- 1-phényl-1-(2-p-méthoxylbenzoylhydrazono)éthane,
- 1-phényl-1-(2-p-diméthylaminobenzoylhydrazono)éthane,
- éthyl-2-(2-benzoylhydrazono)propionate
- méthyl-2-(2-benzoylhydrazono)butyrate
- méthyl-2-(2-benzoylhydrazono)valérate
- méthyl-2-phényl-2-(2-benzoylhydrazono)acétate

L'invention inclut également les semi-carbazones de formule ($Vb_4$).

On peut également citer comme substrats de départ, les cétoimines cycliques avec liaison endo ou exocyclique et plus particulièrement:

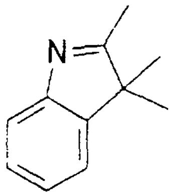 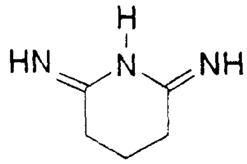 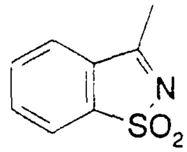

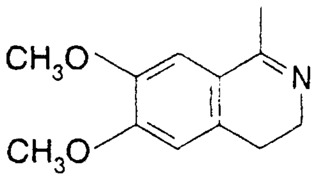 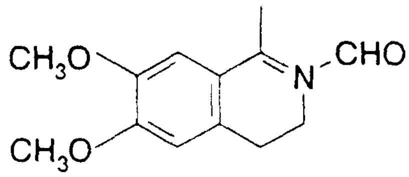 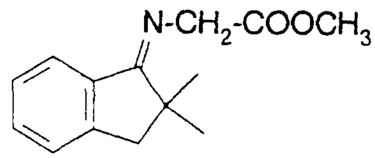

Conformément au procédé de l'invention, l'hydrogénation asymétrique sélective est effectuée en utilisant comme catalyseurs les complexes métalliques de l'invention ligandés par les diphosphines optiquement actives de formule générale (Ia) ou (Ib).

Quand l'invention s'applique plus précisément aux composés cétoniques et plus particulièrement aux composés de formule (Va), on choisit de préférence, de mettre en oeuvre des complexes diphsophine/ruthénium. Dans le cas où il s'agit d'un substrat de type azoté (Vb), notamment une imine, on fait appel à des complexes diphosphine/rhodium ou iridium et plus préférentiellement phosphine/iridium.

En choisissant l'un des isomères optiques de la diphosphine ayant un pouvoir rotatoire (+) ou (-), et en utilisant un complexe diphosphine-métal de transition comprenant l'isomère choisi, le composé cétonique et dérivé est hydrogéné en un composé ayant la configuration absolue désirée.

L'hydrogénation s'effectue généralement à une température comprise entre 20 et 100°C.

La pression d'hydrogène peut être comprise entre 0,1 et 200 bar, et plus préférentiellement entre 1 et 150 bar.

Le complexe diphosphine/métal de transition est utilisé de telie manière que le rapport entre le nombre d'atomes de métal présent dans le complexe et le nombre de moles du composé à hydrogéner soit compris entre 0,1 et 0,0001.

Le procédé d'hydrogénation est mis en oeuvre, de préférence, dans un solvant organique. On fait appel à n'importe quel solvant dans la mesure où il est stable dans les conditions réactionnelles.

On a recours de préférence, à un solvant organique polaire et plus particulièrement aux solvants suivants :

- les éther-oxydes aliphatiques, cycloaliphatiques ou aromatiques et, plus particulièrement, le diéthyléther, le dipropyléther, le diisopropyléther, le dibutyléther, le méthyltertiobutyléther, le ditertiobutyléther, le diméthyléther de l'éthylèneglycol, le diméthyléther du diéthyièneglycol ; le diphényléther, le dibenzyléther, l'anisole, le phénétole, le 1,4-diméthoxybenzène, le vératrole ; le 1,4-dioxane, le tétrahydrofurane (THF),
- les alcools mono- ou polyhydroxylés et plus particulièrement, les monoalcools aliphatiques tels que le méthanol, l'éthanol, le propanol, le butanol, le sec-butanol, le tert-butanol, le pentanol, l'hexanol ; les dialcools aliphatiques tels que l'éthylène glycol, le diéthylène glycol, le propylène glycol ; les alcools cycloaliphatiques tels que le cyclopentanol, le cyclohexanol,
- les cétones aliphatiques telles que l'acétone, la méthyéthylcétone, la diéthylcétone,
- les esters aliphatiques tels que notamment l'acétate de méthyle, l'acétate d'éthyle, l'acétate de propyle.

La concentration du substrat dans le solvant organique varie avantageusement entre 0,01 et 1 mol/l.

On peut éventuellement ajouter, après la formation du complexe d'hydrogénation, un composé basique.

Ce composé basique peut être une base alcaline telle que l'hydroxyde de sodium ou de potassium ou bien une amine primaire, secondaire ou tertiaire, et plus particulièrement, la pyridine, la pipéridine, la triéthylamine et de préférence, la triéthylamine.

La quantité de base ajoutée est telle que le rapport entre le nombre de moles de base et le nombre d'atomes métalliques présent dans le complexe diphosphine/métal de transition, soit compris entre 0 et 25, de préférence, entre 0 et 12.

Dans le cas où l'on met en oeuvre une cétone simple, il peut être souhaitable, afin d'augmenter sa réactivité, d'ajouter une amine chirale, de préférence, une diamine primaire et une base telle que précitée et plus particulièrement la potasse. Comme exemples plus particuliers de diamines, on peut mentionner le 1,1-diphényl-1,2-diaminoéthane,

le 1,1-bis(4-méthoxyphényl)-2-méthyl-1,2-diaminoéthane, le 1,1-bis(4-méthoxyphényl)-2-isopropyl-1,2-diaminoéthane, 1,1-bis(4-méthoxyphényl)-2-isobutyl-1,2-diaminoéthane,

On peut se référer à l'article de Ryoji Noyori et al [J. Am. Chem. Soc. 117, p. 2675 (1995)].

La quantité de base représente généralement 0,5 % en mole de substrat et celle de la diamine de 0,2 à 0,5 % en mole.

On donne ci-après un mode de réalisation préférentiel du procédé de l'invention.

On met en oeuvre ledit procédé dans un autoclave que l'on purge à l'aide d'un gaz inerte, de préférence, l'azote. On charge de préférence, le substrat en solution dans le solvant organique, puis le catalyseur également en solution dans le solvant organique.

On remplace l'azote par l'hydrogène.

L'hydrogénation est terminée lorsque la pression d'hydrogène devient stable.

Le procédé d'hydrogénation selon l'invention permet d'accéder aux différents énantiomères de nombreux dérivés. Ainsi, selon le substrat, on obtient différents produits tels que des alcools à partir des composés de formule (Va), des hydroxylamines, amines, hydrazines ou semi-carbazines à partir respectivement des composés de formule ($Vb_1$), ($Vb_2$), ($Vb_3$), ($Vb_4$).

Les exemples suivants, donnés à titre non limitatif, illustrent la présente invention.

EXEMPLES

Le ligand qui est utilisé dans les complexes métalliques est préparé selon le mode opératoire suivant :

Phospholyllithium :

Dans un ballon de 250 ml, on introduit 11,3 g (0,06 mol) de 1-phényl-3,4-diméthylphosphole, 0,8 g de lithium métal et 100 ml de tétrahydrofurane distillé.

Le mélange est agité sous argon pendant 2 heures, dans un bain d'eau froide.

La solution devient brune.

L'apparition du phospholyllithium est contrôlée par $RMN^{31}P$.

$$RMN^{31}P = \delta(THF) = 55{,}8 \text{ ppm}.$$

Afin de piéger le phényllithium, on ajoute 2,7 g de chlorure d'aluminium à 0°C.

On laisse réagir 30 minutes à 0°C.

1-1' bisphosphole :

Au mélange précédent, on ajoute à température ambiante goutte à goutte, 6 g (0,025 mol) de diiode en solution dans 25 ml de tétrahydrofurane.

Lorsque 90 % de cette solution sont introduits, on vérifie par $RMN^{31}P$, la disparition du phospholylithium.

$$RMN^{31}P = \delta(THF) = -22{,}4 \text{ ppm}.$$

On extrait sous azote le 1-1' bisphosphole du milieu, à l'aide d'hexane.

Bis-[1-phospha-2,3 diphényl-4.5-diméthylnorbornadiène] : (I m) et (I r)

La solution précédente est évaporée à sec, à l'abri de l'air et portée à 140°C.

On introduit alors 8 g de diphénylacétylène et on laisse réagir pendant 15 à 20 minutes.

La disparition du 1-1' bisphosphole est une nouvelle fois suivie par $RMN^{31}P$.

Le spectre est composé de 2 singulets correspondants aux deux diastéréoisomères.

Le produit est extrait à l'éther et lavé à l'eau.

Les phases organiques sont rassemblées puis évaporées à sec.

Le résidu est alors purifié par chromatographie sur colonne de silice (élution à l'hexane pour éliminer le diphénylacétylène en excès puis avec un mélange hexane/dichlorométhane : 80/20 en volume).

Le rendement global est de 30 %.

Complexe de Palladium II avec (I m) et (I r) dénommé (VI m) et (VI r)

Dans un ballon de 500 ml, on introduit 5 g (8,25 mmol) de (I m) et de (I r) que l'on dissout dans 200 ml de dichlorométhane.

On ajoute alors goutte à goutte 3 g (8,25 mmol) de $PdCl_2(PhCN)_2$ dans 100 ml de dichlorométhane.

La réaction conduite sous argon, est immédiate.

La solution est évaporée à sec et le résidu est soumis à une chromatographie sur silice afin de séparer les deux diastéréoisomères.

On élue à l'aide de dichlorométhane pour éliminer les impuretés, puis d'un mélange de dichlorométhane et d'acétate d'éthyle : 95/5 en volume pour séparer le racémique et enfin d'un mélange dichlorométhane/acétate d'éthyle : 80/20 en volume pour séparer le méso.

$$RMN^{31}P = \delta(CH_2Cl_2) = 81{,}9 \text{ ppm}$$ - isomère minoritaire correspondant au racémique.

$$RMN^{31}P = \delta(CH_2Cl_2) = 88{,}1 \text{ ppm}$$ - isomère majoritaire correspondant au méso.

Décomplexation de (VI r)

Dans un ballon de 100 ml, on introduit 1,5 g (0,002 mol) de (VI r) racémique et 20 ml de dichlorométhane.

On ajoute alors 0,5 g de cyanure de sodium et quelques millitres d'eau (3 ml).

On agite vigoureusement sous argon pendant 10 à 15 minutes.

Le bis-[1-phospha-2,3 diphényl-4,5-diméthylnorbornadiène] (I r) est alors extrait avec du dichlorométhane.

La phase organique est lavée à l'eau puis séchée sur sulfate de sodium.

On récupère ainsi (I r) pur.

Le rendement global de la séparation des diastéréoisomères est de 90 %.

La caractérisation du mélange racémique (Ir) est la suivante :

$$RMN^{31}P = \delta(CDCl_3) = -13{,}2 \text{ ppm.}$$

$RMN^1H = \delta(CDCl_3) = 1{,}31$(s, 6H, $CH_3$) ; 1,69(s, 6H, $CH_3$) ; 2,02-2,20 (m, 4H, $CH_2$ pont) ; 6,86-7,29 (m, 20H, phényls).

Complexe binucléaire de palladium II :

Dans 12 ml de benzène, on introduit sous azote 290 mg (0,5 mmol) de (I r) racémique et 300 mg (0,5 mmol) de (+)-di-$\mu$-chloro-bis[(S)-N,N,-diméthyl-$\alpha$-phényléthylamine-2C,N]-dipalladium II.

La complexation est rapide et suivie par $RMN^{31}P$.

La solution brune est évaporée à sec et le résidu chromatographié pour séparer les deux diastéréoisomères (élution toluène/acétate d'éthyle : 80/20 en volume).

On récupère ainsi les deux énantiomères isolés purs sous forme de deux complexes diastéréoisomères de formule :

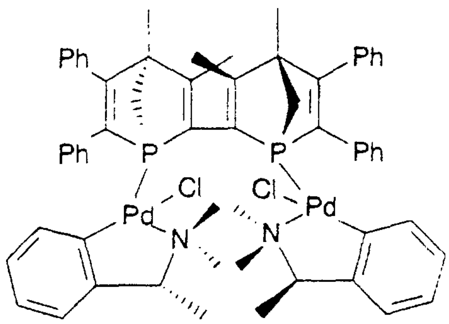

et

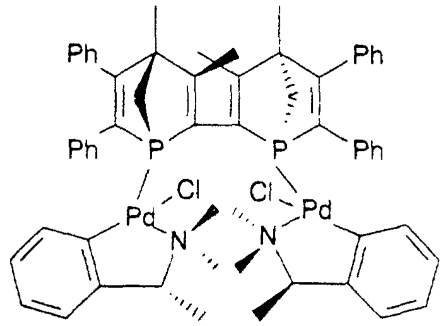

Ces énantiomères sont recouvrés purs en décomplexant comme pour (VI r).

On identifie les diphosphines de formule (Ia) et (Ib) respectivement comme suit:

$$RMN^{31}\ P = \delta(CDCl_3) = -13{,}2\ ppm - [\alpha]_D = +231\ ° \ (c = 1, C_6D_6).$$

$$RMN^{31}P = \delta(CDCl_3) = -13{,}2\ ppm - [\alpha]_D = -198° \ (c = 1, C_6D_6)$$

(avec un $[\alpha]_D$ déterminé pour une concentration de 10 mg/ml et à température ambiante).

Les diphosphines sont utilisées comme ligands dans les exemples qui suivent :

<u>Exemple 1 :</u>

Dans cet exemple, on décrit la préparation d'un complexe de formule $[RuBr_2(P*P)]$.

Dans un schlenk de 10 ml, sous argon, et à température ambiante, on dissout 7,5 mg de phosphine de formule (Ia) (0,013 mmol) et 4 mg du complexe commercial $[Ru(COD)(me\text{-}allyl)_2]$ (0,013 mmol) dans 2 ml d'acétone.

On ajoute ensuite goutte à goutte, 0,11 ml d'une solution d'acide bromhydrique 0,29 M dans du méthanol (0,026 mmol).

On agite 30 min. La solution d'abord incolore, devient marron.

Le composé obtenu après évaporation du solvant est utilisé sans purification.

$$RMN\ ^{31}P\ (acétone) : \delta_A = 97{,}0\ ppm\ \delta_B = 87{,}0\ ppm$$

$$^3J_{AB} = 21{,}0\ Hz$$

<u>Exemple 2</u>

Dans cet exemple, on effectue l'hydrogénation asymétrique, à l'aide du catalyseur de l'exemple 1 du composé suivant :

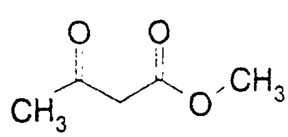

Dans un schlenk, on dissout 0,1 ml dudit composé dans 3 ml de méthanol.

On prépare ensuite comme proposé plus haut, le complexe de l'exemple 1.

On évapore l'acétone et on dissout le résidu dans 2 ml de méthanol.

On introduit alors les deux solutions dans un autoclave préalablement purgé et maintenu sous atmosphère d'azote.

Puis, on introduit l'hydrogène jusqu'à une pression de 4 atmosphères.

On agite à 20°C pendant 48 heures.
On vide l'hydrogène en excès et on récupère la solution de la réaction.
Le solvant est évaporé et le résidu analysé en $RMN^1H$ pour vérifier l'avancement de la réaction.
On obtient le 3-hyroxybutanoate de méthyle. La réaction est quantitative.
L'excès énantiomérique est déterminé par chromatographie gazeuse chirale.
Avec la diphosphine (la), ee = 80 %.

Exemple 3:

Dans cet exemple, on décrit la préparation d'un complexe de formule $RuBr_2$(P*P) dans laquelle (P*P) représente la diphosphine de formule (Ib).
La préparation dudit complexe est préparé selon le mode opératoire de l'exemple 1.

Exemple 4 :

Dans cet exemple, on effectue l'hydrogénation asymétrique, à l'aide du catalyseur de l'exemple 3, du composé suivant :

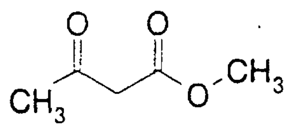

La mise en oeuvre est la même que dans l'exemple 2.
Avec la disphosphine (Ib), ee = 81 %.

Exemple 5:

Dans cet exemple, on effectue l'hydrogénation asymétrique à l'aide du catalyseur de l'exemple 1 du composé suivant:

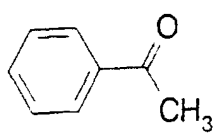

Dans un schlenk de 10 ml, sous argon, on dissout 780 mg (6,5 mmol) de cétone dans 7 ml d'isopropanol.
On y ajoute 2,75 mg de diamine chirale commerciale (S,S)-1,2-diphényl-1,2-éthanediamine, et 1,45 mg de potasse.
On agite rigoureusement cette solution jusqu'à ce que le mélange soit homogène.
De même, on dissout le catalyseur de l'exemple 1 dans 2 ml d'isopropanol.
Les deux solutions sont introduites à l'aide d'une seringue dans l'autoclave.
On ajoute l'hydrogène jusqu'à pression de 5 atm.
On agite le mélange pendant 15 h à 25°C.
On vide l'hydrogène en excès et l'on récupère la solution de la réaction.
Après évaporation du solvant, l'avancement de la réaction est contrôlée par $RMN^1H$ du produit brut.

- TT = 45 %.

L'alcool est ensuite purifié par distillation sous vide à l'appareil à boules (Kugelröhr®).
La configuration de l'alcool est déterminée par le signe du pouvoir rotataire $[\alpha]_D>0$ (c = 1, éther).
L'excès énantiomérique est déterminé par chromatographie chirale CLHP sur une colonne Daicel, Chiracel OD (n-hexane/éthanol - 95/5 ; 0,5 ml/min) ;

- $t_R$ (S)-1-phényléthanol = 14,6 min ; (t = temps de rétention)
- $t_R$ (R)-1-phényléthanol = 17,0 min ;
- ee = 57 % (R).

Exemple 6 :

Dans cet exemple, on effectue l'hydrogénation asymétrique à l'aide du catalyseur de l'exemple 1 du composé suivant :

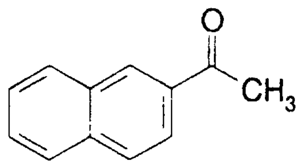

Dans un schlenk, on dissous 1,098 g dudit composé dans 7 ml d'isopropanol.
On procède ensuite comme dans l'exemple 5.
L'hydrogénation est effectuée pendant 10 h.
Le rendement calculé par $RMN^1H$ est de 60 %.
L'excès énantiomérique est déterminé par $RMN^1H$ de l'ester formé avec l'acide commercial (R)-(+)-α-méthoxytrifluorométhylphénylacétique (MTPA).
A l'abri de l'air, on ajoute à 50 mg de l'alcool dissout dans 4 ml de dichlorométhane, 70 mg d'acide MTPA, 0,3 ml d'une solution commerciale de 1,3-dicyclohexylcarbodiimide (DCC) 1 M dans le dichlorométhane et 50 mg de p-diméthylaminopyridine.
On agite ce mélange pendant une nuit à température ambiante.
Après évaporation du solvant, l'ester est extrait à l'éther.

ee = 81 % (R)

Exemple 7 :

Dans cet exemple, on décrit la préparation d'un complexe de formule $[Ir(COD)(P*P)]^+X^-$, dans laquelle COD représente le 1.5-cyclooctadiène, P*P représente la diphosphine Ib, $X^-$ l'anion $BPh_4^-$.
Dans un schlenk de 10 ml, on dissout, sous argon, 4,35 mg de précurseur commercial $(Ir(COD)Cl]_2$ dans 1 ml d'un mélange benzène/MeOH (1:1 en volume). On ajoute alors goutte à goutte une solution de 7,5 mg de diphosphine Ib dans 1 ml du même mélange de solvant.
Après 15 min d'agitation, on obtient le complexe attendu.
On évapore le solvant.
La poudre rouge obtenue est lavée 2 fois à l'isopropanol et redissoute dans 1 ml de dichlorométhane.
On additionne alors 1,5 équivalent de $NaBPh_4$ (7,2 mg) commercial.
La solution est concentrée à la pompe à vide (0,2 mm de mercure) et le complexe précipite.

RMN $^{31}P$ $(CD_2Cl_2)$ $\delta$ = 64,5 ppm.

Exemple 8 :

Dans cet exemple, on effectue l'hydrogénation asymétrique à l'aide du catalyseur de l'exemple 7 du composé suivant :

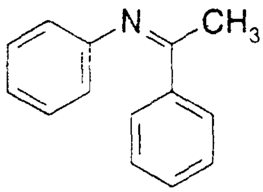

Dans un schlenk, sous argon, on dissout 506 mg dudit composé dans 3 ml d'un mélange de toluène/méthanol (1:1 en volume).
On prépare dans un deuxième schlenk une solution de 3 ml du catalyseur de l'exemple 7 dans le même mélange de solvants. On ajoute, alors le co-catalyseur (10 mg) de $Bu_4N^+I^-$ commercial.
On introduit ensuite consécutivement les deux solutions à l'aide d'une seringue dans un autoclave préalablement purgé et maintenu sous atmosphère d'azote.
Puis, on introduit 2 fois l'hydrogène jusqu'à une pression de 3 atm. pour purger l'autoclave.
Enfin, on pressurise à 20 atm.
On agite pendant 48 h à 25°C.
Après évaporation du solvant, l'avancement de la réaction de 50 % est contrôlé par $RMN^1H$ du produit brut.
Ce dernier est purifié par distillation et l'excès énantiomérique est évalué par la mesure du pouvoir rotatoire ee = 20 % (S).

**Revendications**

1. Procédé d'hydrogénation asymétrique d'un composé cétonique et dérivé **caractérisé par le fait que** l'on effectue l'hydrogénation asymétrique dudit composé, en présence d'une quantité efficace d'un complexe métallique comprenant à titre de ligand, une diphosphine optiquement active répondant à l'une des formules suivantes :

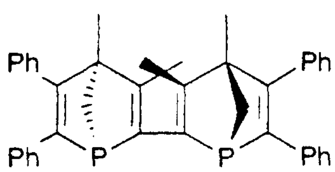

(Ia) - (S,S) - (+)

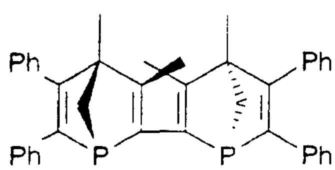

(Ib) - (R,R) - (-)

2. Procédé selon la revendication 1 **caractérisé par le fait que** le complexe métallique comprend und diphosphine optiquement active de formule (Ia) ou (Ib) et un métal de transition choisi parmi : le rhodium, le ruthénium, le rhénium, l'iridium, le cobalt, le nickel, le platine, le palladium.

3. Procédé selon l'une des revendications 1 et 2 **caractérisé par le fait que** le complexe métallique répond à l'une des formules suivantes :

$$[M\ L_2(P^*P)]\ Y \qquad (IIa)$$

$$[M\ L_2(P^*P]\ Y \qquad (IIb)$$

dans lesdites formules :

- (P*P) représente dans la formule (IIa) la diphosphine de formule (Ia) et dans la formule (IIb) la diphosphine de formule (Ib),
- M représente le rhodium ou l'iridium,
- Y représente un ligand anionique coordinant,
- L représente un ligand neutre.

4. Procédé selon la revendication 3 **caractérisé par le fait que** le complexe répond à la formule (IIa) ou (IIb) dans laquelle :

- L représente une oléfine ayant de 2 à 12 atomes de carbone et deux ligands L peuvent être liés entre eux pour former une chaîne hydrocarbonée polyinsaturée, linéaire ou cyclique ; L représentant de préférence, le 1,5-cyclooctadiène, le norbornadiène, l'éthylène,
- Y représente un anion $PF_6^-$, $PCl_6^-$, $BF_4^-$, $BCl_4^-$, $SbF_6^-$, $SbCl_6^-$, $BPh_4^-$, $ClO_4^-$, CN-, $CF_3SO_3^-$, halogène de préférence, Cl- ou $Br^-$, un anion 1,3-dicétonate, alkylcarboxylate, haloalkylcarboxylate avec un radical alkyle inférieure, un anion phénylcarboxylate ou phénolate dont le cycle benzénique peut être substitué par des radicaux alkyle inférieurs et/ou des atomes d'halogène.

5. Procédé selon l'une des revendications 1 et 2 **caractérisé par le fait que** le complexe métallique répond à l'une des formules suivantes :

$$[Ir\ L\ (P^*P)]\ Y \qquad (IIIa)$$

$$[Ir\ L\ (P^*P]\ Y \qquad (IIIb)$$

dans lesdites formules, (P*P), L et Y ayant les significations données pour les formules (IIa) et (IIb) dans les revendications 3 et 4.

6. Procédé selon l'une des revendications 1 et 2 **caractérisé par le fait que** le complexe métallique répond à l'une des formules suivantes :

$$[RuY_1Y_2(P^*P)] \qquad (IVa)$$

$$[RuY_1Y_2(P^*P)] \qquad (IVb)$$

dans lesdites formules :

- (P*P) représente dans la formule (IVa) la diphosphine de formule (Ia) et dans la formule (IVb) la diphosphine de formule (Ib),
- $Y_1$ et $Y_2$, identiques ou différents, représentent de préférence, un anion $PF_6^-$, $PCl_6^-$, $BF_4^-$, $BCl_4^-$, $SbF_6^-$, $SbCl_6^-$, $BPh_4^-$, $ClO_4^-$, $CF_3SO_3^-$, un atome d'halogène, plus particulièrement, chlore ou brome ou un anion carboxylate, préférentiellement, acétate, trifluoroacétate.

7. Procédé selon l'une des revendications 1 et 2 **caractérisé par le fait que** le complexe métallique répond à l'une des formules suivantes :

$$[RuY_1Ar(P^*P)Y_2] \qquad (IVc)$$

$$[RuY_1Ar(P^*P)Y_2] \qquad (IVd)$$

dans lesdites formules :

- (P*P) représente dans la formule (IVc) la diphosphine de formule (Ia) et dans la formule (IVd) la diphosphine de formule (Ib),
- Ar représente le benzène, le p-méthylisopropylbenzène, l'hexaméthylbenzène,
- $Y_1$ représente un atome d'halogène, de préférence, chlore ou brome,
- $Y_2$ représente un anion de préférence, $PF_6^-$, $PCl_6^-$, $BF_4^-$, $BCl_4^-$, $SbF_6^-$, $SbCl_6^-$, $BPh_4^-$, $ClO_4^-$, $CF_3SO_3^-$.

**8.** Procédé selon l'une des revendications 1 à 7 **caractérisé par le fait que** le composé cétonique et dérivé répond à la formule générale (V):

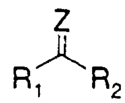
(V)

dans ladite formule (V) :

- $R_1$ est différent de $R_2$,
- $R_1$ et $R_2$ représentent un radical hydrocarboné ayant de 1 à 30 atomes de carbone comprenant éventuellement un ou plusieurs groupes fonctionnels,
- $R_1$ et $R_2$, peuvent former un cycle comprenant éventuellement un autre hétéroatome,
- Z est ou comprend un hétéroatome, oxygène ou azote ou un groupe fonctionnel comprenant au moins un de ces hétéroatomes.

**9.** Procédé selon la revendication 8 **caractérisé par le fait que** le composé cétonique et dérivé répond à la formule générale (V), dans laquelle les radicaux $R_1$ et $R_2$ représentent un radical hydrocarboné monovalent, substitué ou non qui peut être un radical aliphatique acyclique saturé ou insaturé, linéaire ou ramifié ; un radical carbocyclique ou hétérocyclique saturé, insaturé ou aromatique, monocyclique ou polycyclique.

**10.** Procédé selon l'une des revendications 8 et 9 **caractérisé par le fait que** le composé cétonique et dérivé répond à la formule (Va) :

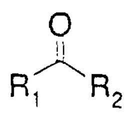
(Va)

dans ladite formule (Va) :

- $R_1$ est différent de $R_2$, les radicaux $R_1$ et $R_2$ représentent un radical hydrocarboné ayant de 1 à 30 atomes de carbone comprenant éventuellement une autre fonction cétone et/ou acide, ester, thioacide, thioester,
- $R_1$ et $R_2$ peuvent former un cycle carbocyclique ou hétérocyclique, substitué ou non, ayant de 5 à 6 atomes.

**11.** Procédé selon la revendication 10 **caractérisé par le fait que** le composé cétonique est une cétone simple.

**12.** Procédé selon l'une des revendications 10 et 11 **caractérisé par le fait que** le composé cétonique répond à la formule ($Va_1$):

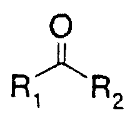
($Va_1$)

dans ladite formule ($Va_1$):

- $R_1$ et $R_2$ représentent :

  . un radical alkyle, linéaire ou ramifié, ayant de 1 à 12 atomes de carbone, de préférence de 1 à 4 atomes de carbone, tel que méthyle, éthyle, propyle, isopropyle, butyle, isobutyle, sec-butyle, tert-butyle,
  . un radical alcényle ayant de 2 à 12 atomes de carbone, de préférence, un radical allyle,
  . un radical phényle, naphtyle ou benzyle éventuellement substitué,
  . un radical triphénylméthyle,
  . un radical de formule :

$$-R_3-OH$$

$$-R_3-O-R_4$$

$$-R_3-CO-R_4$$

$$-R_3-COOR_4$$

$$-R_3-CHO$$

$$-R_3-NO_2$$

$$-R_3-CN$$

$$-R_3-N(R_4)_2$$

$$-R_3-CO-N(R_4)_2$$

$$-R_3-PO-(OR_4)_2$$

$$-R_3-SH$$

$$-R_3-X$$

$$-R_3-CF_3$$

  dans lesdites formules, $R_3$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; les radicaux $R_4$, identiques ou différents, représentent un atome d'hydrogène ou un radical alkyle linéaire ou ramifié ayant de 1 à 6 atomes de carbone, un radical benzyle ou un radical phényle ; X symbolise un atome d'halogène, de préférence un atome de chlore, de brome ou de fluor,

- $R_1$ et $R_2$ peuvent former un cycle carbocyclique ou hétérocyclique, substitué ou non, ayant de 5 à 6 atomes.

**13.** Procédé selon la revendication 12 **caractérisé par le fait que** le composé cétonique de formule ($Va_1$) est choisi

parmi :

- méthylphénylcétone,
- isopropylphénylcétone,
- cyclopropylphénylcétone,
- allylphénylcétone,
- p-méthylphénylméthylcétone,
- benzylphénylcétone,
- phényltriphénylméthylcétone,
- o-bromoacétophénone,
- α-bromoacétone,
- α-dibromoacétone,
- α-chloroacétone,
- α-dichloroacétone,
- α-trichloroacétone,
- 1-chloro-3,3-dichloroacétone,
- 1-chloro-2-oxobutane,
- 1-fluoro-2-oxobutane,
- 1-chloro-3-méthyl-2-butanone,
- α-chloroacétophénone,
- 1-chloro-3-phénylacétone,
- α-méthylaminoacétone,
- α-diméthylaminoacétone,
- 1-butylamino-2-oxopropane,
- 1-dibutylamino-2-oxopropane.
- 1-méthylamino-2-oxobutane,
- 1-diméthylamino-2-oxobutane,
- 1-diméthylamino-3-méthyl-2-oxobutane,
- 1-diméthylamino-2-oxopentane,
- α-diméthylaminoacétophénone,
- α-hydroxyacétone,
- 1-hydroxy-3-méthyl-2-butanone,
- 1-hydroxy-2-oxobutane,
- 1-hydroxy-2-oxopentane,
- 1-hydroxy-2-oxohexane,
- 1-hydroxy-2-oxo-3-méthylbutane,
- α-hydroxyacétophénone,
- 1-hydroxy-3-phénylacétone,
- α-méthoxyacétone,
- α-méthoxyacétophénone,
- α-méthoxyacétophénone,
- α-éthoxyacétone,
- α-butoxyacétophénone,
- α-chloro-p-méthoxyacétophénone,
- α-naphténone,
- 1-éthoxy-2-oxobutane,
- 1-butoxy-2-oxobutane,
- α-diméthoxyphosphorylacétone,
- 3-oxotétrahydrothiophène.

**14.** Procédé selon la revendication 10 **caractérisé par le fait que** le composé cétonique est une dicétone.

**15.** Procédé selon l'une des revendications 10 et 14 **caractérisé par le fait que** composé cétonique répond à la formule ($Va_2$):

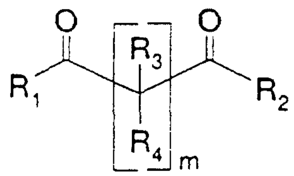

(Va$_2$)

dans ladite formule (Va$_{2)}$:

- m est égal à 0,1,2 ou 3, de préférence, égal à 0 ou 1,
- les radicaux $R_1$ et $R_2$ différents, représentent :

  . un radical alkyle, linéaire ou ramifié, ayant de 1 à 12 atomes de carbone, éventuellement porteur d'un atome d'halogène, de préférence de 1 à 4 atomes de carbone, tel que méthyle, éthyle, propyle, isopropyle, butyle, isobutyle, sec-butyle, tert-butyle,
  . un radical alcényle ayant de 2 à 12 atomes de carbone, de préférence, un radical allyle,
  . un radical phényle, naphtyle ou benzyle éventuellement substitué,
  . un radical de formule :

$$-R_5-OH$$

$$-R_5-O-R_6$$

$$-R_5-CO-R_6$$

$$-R_5-COOR_6$$

$$-R_5-N(R_6)_2$$

$$-R_5-CO-N(R_6)_2$$

$$-R_5-PO-(OR_6)_2$$

$$-R_5-SH$$

$$-R_5-X$$

$$-R_5-CF_3$$

  dans lesdites formules, $R_5$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; les radicaux $R_6$, identiques ou différents, représentent un atome d'hydrogène ou un radical alkyle linéaire ou ramifié ayant de 1 à 6 atomes de carbone, un radical benzyle ou un radical phényle ; X symbolise un atome d'halogène, de préférence un atome de chlore, de brome ou de fluor,
  . l'un des radicaux $R_1$ et $R_2$ pouvant représenter un atome d'hydrogène,

- les radicaux $R_3$ et $R_4$, identiques ou différents, représentent :

  . un atome d'hydrogène,
  . un radical alkyle, linéaire ou ramifié, ayant de 1 à 12 atomes de carbone, éventuellement porteur d'un atome d'halogène, de préférence de 1 à 4 atomes de carbone, tel que méthyle, éthyle, propyle, isopropyle, butyle, isobutyle, sec-butyle, tert-butyle,
  . un atome d'halogène,
  . un groupe du type $-R_5-COOR_6$ dans lequel $R_5$ et $R_6$ ayant la signification donnée précédemment,

- $R_1$ ou $R_2$ et $R_3$ ou $R_4$ peuvent former un cycle carbocyclique ou hétérocyclique, substitué ou non, ayant de 5 à 6 atomes.

**16.** Procédé selon la revendication 15 **caractérisé par le fait que** le composé cétonique de formule ($Va_2$) est choisi parmi :

- α-formylacétone,
- diacétyle,
- 3,4-dioxohexane,
- 4,5-dioxooctane,
- 1-phényl-1,2-dioxopropane,
- 1-phényl-2,3-dioxobutane,
- dibenzoyle,
- p-méthoxydibenzoyle
- 1,2-cyclopentanedione,
- 1,2-cyclohexanedione,
- acétylacétone,
- 3,5-heptanedione,
- 4,6-nonanedione,
- 5,7-undecadione,
- 2,4-hexanedione,
- 2,4-heptanedione,
- 2,4-octanedione,
- 2,4-nonanedione,
- 3,5-nonanedione,
- 3,5-décanedione,
- 2,4-dodécanedione,
- 1-phényl-1,3-butanedione,
- 1-phényl-1,3-pentanedione,
- 1-phényl-1,3-hexanedione,
- 1-phényl-1,3-heptanedione,
- 3-méthyl-2,4-pentanedione,
- 1,3-diphényl-1,3-propanedione,
- 1,5-diphényl-2,4-pentanedione,
- 1,3-di(trifluorométhyl)-1,3-propanedione,
- 3-chloro-2,4-pentanedione
- 1,5-dichloro-2,4-pentanedione,
- 1,5-dihydroxy-2,4-pentanedione,
- 1,5-dibenzyloxy-2,4-pentanedione,
- 1,5-diamino-2,4-pentanedione,
- 1,5-di(méthylamino)2,4-pentanedione,
- 1,5-di(diméthylamino)-2,4-pentanedione,
- 3,5-dioxo-hexanoate de méthyle.
- 3-carbométhoxy-2,4-pentanedione,
- 3-carboéthoxy-2,4-pentanedione,
- 1,3-cyclopentanedione,
- 1,3-cyclohexanedione,
- 1,3-cycloheptanedione,
- 5-carboéthoxy-1,3-cyclopentanedione,

- 2-acétyl-1-cyclopentanone,
- 2-acétyl-1-cyclohexanone.

**17.** Procédé selon la revendication 10 **caractérisé par le fait que** le composé cétonique est cétoacide, céthioacide ou dérivé.

**18.** Procédé selon l'une des revendications 10 et 17 **caractérisé par le fait que** le composé cétonique répond à la formule ($Va_3$) ou ($Va_4$):

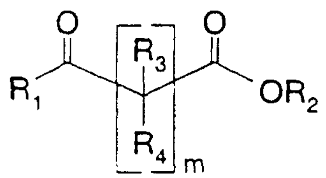

($Va_3$) ou

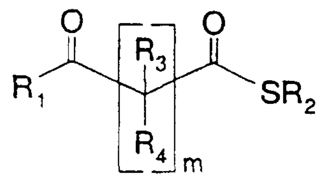

($Va_4$)

dans lesdites formules ($Va_3$) ou ($Va_4$) :

- m est égal à 0,1,2 ou 3, de préférence, égal à 0 ou 1,
- le radical $R_1$ représente :
  . un radical alkyle, linéaire ou ramifié, ayant de 1 à 12 atomes de carbone, éventuellement porteur d'un atome d'halogène, de préférence de 1 à 4 atomes de carbone, tel que méthyle, éthyle, propyle, isopropyle, butyle, isobutyle, sec-butyle, tert-butyle,
  . un radical alcényle ayant de 2 à 12 atomes de carbone, de préférence, un radical allyle,
  . un radical phényle, naphtyle ou benzyle éventuellement substitué,
  . un radical de formule :

$$-R_5-OH$$

$$-R_5-O-R_6$$

$$-R_5-CO-R_6$$

$$-R_5-COOR_6$$

$$-R_5-N(R_6)_2$$

$$-R_5-CO-N(R_6)_2$$

$$-R_5-PO-(OR_6)_2$$

$$-R_5-SH$$

$$-R_5-X$$

$$-R_5-CF_3$$

dans lesdites formules, $R_5$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; les radicaux $R_6$, identiques ou différents, représentent un atome d'hydrogène ou un radical alkyle linéaire ou ramifié ayant de 1 à 6 atomes de carbone, un radical benzyle ou un radical phényle ; X symbolise un atome d'halogène, de préférence un atome de chlore, de brome ou de fluor,

- le radical $R_2$ représente :

  . un atome d'hydrogène,
  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  . un radical benzyle.
  . un radical phényle,

- les radicaux $R_3$ et $R_4$, identiques ou différents, représentent :

  . un atome d'hydrogène,
  . un radical alkyle, linéaire ou ramifié, ayant de 1 à 12 atomes de carbone, de préférence de 1 à 4 atomes de carbone, tel que méthyle, éthyle, propyle, isopropyle, butyle, isobutyle, sec-butyle, tert-butyle,
  . un atome d'halogène,
  . un groupe du type $-R_5-COOR_6$ dans lequel $R_5$ et $R_6$ ayant la signification donnée précédemment,
  . un groupe du type $-R_5-CO-N(R_6)_2$ dans lequel $R_5$ et $R_6$ ayant la signification donnée précédemment,

- $R_1$ et $R_2$ ou $R_1$ et $R_3$ ou $R_1$ et $R_4$ ou $R_2$ et $R_4$ peuvent former un cycle carbocyclique ou hétérocyclique, substitué ou non, ayant de 5 à 6 atomes.

**19.** Procédé selon la revendication 18 **caractérisé par le fait que** le composé cétonique de formule ($Va_3$) ou ($Va_4$) est choisi parmi :

- acide 2-acétylbenzoïque,
- acide pyruvique,
- acide 2-oxobutanoïque,
- acide 3-méthyl-2-oxobutanoïque,
- acide phénylglyoxylique,
- acide phénylpyruvique,
- acide p-méthoxyphényipyruvique,
- acide 3,4-diméthoxyphénylpyruvique,
- acétoacétate de méthyle,
- acétoacétate d'éthyle,
- acétoacétate de n-propyle,
- acétoacétate d'isopropyle,
- acétoacétate de n-butyle,
- acétoacétate de t-butyle,
- acétoacétate de n-pentyle,
- acétoacétate de n-hexyle,
- acétoacétate de n-heptyle,
- acétoacétate de n-octyle,
- 3-oxopentanoate de méthyle,
- 3-oxohexanoate de méthyle,
- 3-oxoheptanoate de méthyle,
- 3-oxooctanoate d'éthyle,
- 3-oxononanoate d'éthyle,
- 3-oxodécanoate d'éthyle,
- 3-oxoundécanoate d'éthyle,
- 3-oxo-3-phénylpropanoate d'éthyle,
- 4-phényl-3-oxobutanoate d'éthyle,
- 5-phényl-3-oxopentanoate de méthyle,

- 3-oxo-3-p-méthoxyphénylpropanoate d'éthyle,
- 4-chloroacétoacétate de méthyle,
- 4-chloroacétoacétate d'éthyle,
- 4-fluoroacétoacétate de méthyle,
- 3-trifluorométhyl-3-oxopropanoate d'éthyle,
- 4-hydroxy-3-oxobutanoate d'éthyle,
- 4-méthoxyacétoacétate de méthyle,
- 4-tert-butoxyacétoacétate de méthyle,
- 4-benzyloxy-3-oxobutanoate de méthyle,
- 4-benzyloxy-3-oxobutanoate d'éthyle,
- 4-amino-3-oxobutanoate de méthyle,
- 3-méthylamino-3-oxobutanoate d'éthyle,
- 4-diméthylamino-3-oxobutanoate de méthyle,
- 4-diméthylamino-3-oxobutanoate d'éthyle,
- 2-méthylacétoacétate de méthyle,
- 2-méthylacétoacétate d'éthyle,
- 2-chloroacétoacétate d'éthyle,
- 2-acétylsuccinate de diéthyle,
- 2-acétylglutarte de diéthyle,
- acétylmalonate de diméthyle,
- acétoacétate de thiométhyle,
- acétoacétate de thioéthyle,
- acétoacétate de thiophényle,
- pyruvate de méthyle,
- 3-méthyl-2-oxobutanoate d'éthyle,
- phénylglyoxolate d'éthyle,
- phénylpyruvate de méthyle,
- phénylpyruvate d'éthyle,
- 3-oxobutanoic diméthylamide,
- 3-oxobutanoic benzylamide,
- 2-carboéthoxy-cyclopentanone,
- 2-carboéthoxy-cyclohexanone,
- cétopentalactone.
- acide 4-oxopentanoïque,
- acide 4-oxohexanoique,
- acide 4-oxoheptanoique,
- acide 4-oxodécanoîque,
- acide 4-oxododécanoïque,
- acide 4-phényl-4-oxybutyrique,
- acide 4-p-méthoxyphényl-4-oxybutyrique,
- acide 4-(3,4-diméthoxyphényl)-4-oxobutyrique,
- acide 4-(3,4,5-triméthoxyphényl)-4-oxobutyrique,
- acide 4-p-chlorophényl-4-oxybutyrique,
- acide 4-phényl-4-oxobutyrique.

**20.** Procédé selon la revendication 10 **caractérisé par le fait que** le composé cétonique répond à la formule (Va) dans laquelle les radicaux $R_1$ et $R_2$ forment avec l'atome de carbone qui porte le groupe carbonyle un cycle ou plusieurs cycles, de préférence, deux cycles, saturés et/ou insaturés, éventuellement aromatiques, comprenant de 5 à 6 atomes de carbone dans chaque cycle et portant éventuellement un autre groupe carbonyle et/ou un substituant.

**21.** Procédé selon l'une des revendications 8 et 9 **caractérisé par le fait que** le dérivé cétonique répond à la formule (Vb) :

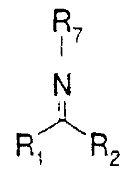 (Vb)

dans ladite formule (Vb) :

- $R_1$ différent de $R_2$, ont la signification donnée précédemment,
- $R_7$ représente :

  . un atome d'hydrogène,
  . un groupe hydroxyle,
  . un groupe $OR_8$
  . un radical hydrocarboné $R_8$,
  . un groupe de formule

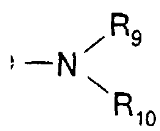

  . un groupe de formule

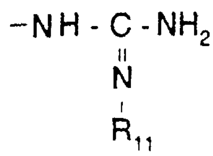

avec $R_8$, $R_9$, $R_{10}$, et $R_{11}$ qui représentent un atome d'hydrogène ou un groupement hydrocarboné ayant de 1 à 30 atomes de carbone.

**22.** Procédé selon la revendication 21 **caractérisé par le fait que** le dérivé cétonique répond à l'une des formules suivantes :

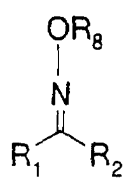 ($Vb_1$)

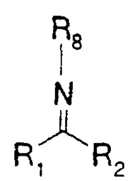 ($Vb_2$)

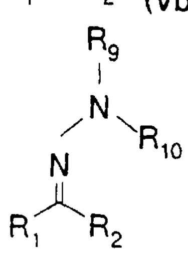 ($Vb_3$)

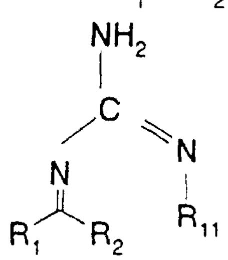 ($Vb_4$)

dans lesdites formules ($Vb_1$) à ($Vb_4$) :

- les radicaux $R_1$ et $R_2$, $R_8$ à $R_{11}$ représentent:

  . un radical alkyle linéaire ou ramifié ayant de 1 à 12 atomes de carbone,
  . un radical cycloalkyle ayant de 5 à 12 atomes de carbone,
  . un radical aryle ayant de 6 à 12 atomes de carbone,
  . un radical aralkyle ayant de 7 à 12 atomes de carbone,
  . un radical aryle ayant de 6 à 12 atomes de carbone porteurs de substituants tels qu'un radical alkyle ou alkoxy ayant de 1 à 4 atomes de carbone, un groupe amino, ($C_1$-$C_4$)alkylamino ou di-($C_1$-$C_4$)alkylamino, un groupe nitro, un atome d'halogène, un groupe ($C_1$-$C_4$)alkoxycarbonyle,
  . un radical aryle ayant de 6 à 12 atomes de carbone,
  . un radical hétérocyclique saturé ou insaturé,
  . un radical alcanoyle ayant de 1 à 12 atomes de carbone,
  . un radical arylcarbonyle ayant de 6 à 12 atomes de carbone,
  . un radical arylalcanoyle ayant de 6 à 12 atomes de carbone,

- $R_1$ et $R_2$ , $R_1$ et $R_8$ , $R_2$ et $R_8$, $R_1$ et $R_9$, $R_2$ et $R_{10}$, $R_1$ et $R_{11}$, $R_2$ et $R_{11}$ peuvent former un cycle carbocyclique ou hétérocyclique, substitué ou non, monocyclique ou polycyclique, ayant de 5 à 6 atomes dans chaque cycle.

**23.** Procédé selon l'une des revendications 21 et 22 **caractérisé par le fait que** le dérivé cétonique répond à l'une des formules ($Vb_1$) à ($Vb_4$) dans lesquelles les radicaux $R_1$ et $R_2$, $R_8$ à $R_{11}$, identiques ou différents représentent :

. un radical alkyle linéaire ou ramifié ayant de 1 à 4 atomes de carbone,
. un radical cyclopentyle ou cyclohexyle,
. un radical phényle
. un radical benzyle ou phényléthyle,
. un radical phényle porteur de substituants tels qu'un radical alkyle ou alkoxy ayant de 1 à 4 atomes de carbone, un groupe amino, ($C_1$-$C_4$)alkylamino ou di-($C_1$-$C_4$)alkylamino, un groupe nitro, un atome d'halogène, un groupe ($C_1$-$C_4$)alkoxycarbonyle,
. un radical naphtyle,
. un radical hétérocyclique oxygéné ou azoté saturé ou insaturé à 5 ou 6 atomes,
. un radical acétyle, un radical benzoyle,
. un radical arylalcanoyle ayant de 6 à 12 atomes de carbone,

**24.** Procédé selon l'une des revendications 21 à 23 **caractérisé par le fait que** le dérivé cétonique est choisi parmi :

- oxime de l'acétophénone
- N-isobutyl-2-iminopropane
- N-isobutyl-1-méthoxy-2-iminopropane
- N-benzyl-1-imino-1-(phényl)éthane
- N-benzyl-1-imino-1-(4-méthoxyphényl)éthane
- N-benzyl-1-imino-1-(2-méthoxyphényl)éthane
- N-phényl-2-iminopentane
- N-(2,6-diméthylphényl)-2-iminopentane
- N-(2,4,6-triméthylphényl)-2-iminopentane
- N-phényl-1-imino-1-phényléthane
- N-phényl-1-méthoxy-2-iminopropane
- N-(2,6-diméthylphényl)-1-méthoxy-2-iminopropane
- N-(2-méthyl-6-éthylphényl)-1-méthoxy-2-iminopropane
- 1-cyclohexyl-1-(2-benzoylhydrazono)éthane,
- 1-phényl-1-(2-benzoylhydrazono)éthane,
- 1-p-méthoxyphényl-1-(2-benzoylhydrazono)éthane,
- 1-p-éthoxyphényl-1-(2-benzoylhydrazono)éthane,
- 1-p-nitrophényl-1-(2-benzoylhydrazono)éthane,
- 1-p-bromophényl-1-(2-benzoylhydrazono)éthane,
- 1-p-carboéthoxyphényl-1-(2-benzoylhydrazono)éthane,
- 1,2-diphényl-1-(2-benzoylhydrazono)éthane,

- 1,2-diphényl-1-(2-benzoylhydrazono)éthane,
- 3-méthyl-2-(2-p-diméthylaminobenzoylhydrazono)butane,
- 1-phényl-1-(2-p-méthoxylbenzoylhydrazono)éthane,
- 1-phényl-1-(2-p-diméthylaminobenzoylhydrazono)éthane,
- éthyl-2-(2-benzoylhydrazono)propionate
- méthyl-2-(2-benzoylhydrazono)butyrate
- méthyl-2-(2-benzoylhydrazono)valérate
- méthyl-2-phényl-2-(2-benzoylhydrazono)acétate
- les cétoimines cycliques avec liaison endo ou exocyclique:

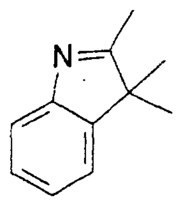 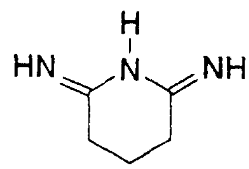 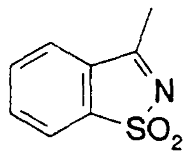

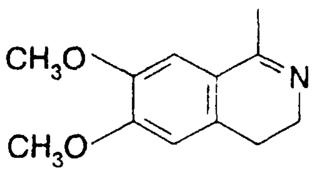 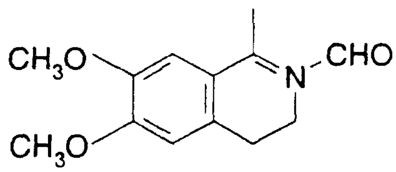 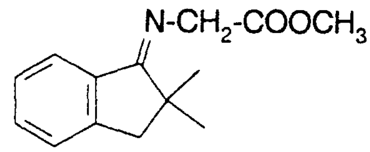

**25.** Procédé selon l'une des revendications 1 à 24 **caractérisé par le fait que** l'hydrogénation s'effectue généralement à une température comprise entre 20 et 100°C.

**26.** Procédé selon l'une des revendications 1 à 25 **caractérisé par le fait que** la pression d'hydrogène peut être comprise entre 0,1 et 200 bar, et plus préférentiellement entre 1 et 150 bar.

**27.** Procédé selon l'une des revendications 1 à 26 **caractérisé par le fait que** l'on met en oeuvre au moins un complexe selon l'une des revendications 2 à 7.

**28.** Procédé selon l'une des revendications 1 à 27 **caractérisé par le fait que** le rapport entre le nombre d'atomes de métal présent dans le complexe et le nombre de moles du composé à hydrogéner est compris entre 0,1 et 0,0001.

**29.** Procédé selon l'une des revendications 1 à 28 **caractérisé par le fait que** l'on ajoute, après la formation du complexe d'hydrogénation, un composé basique, de préférence, la potasse.

**30.** Procédé selon l'une des revendications 1 à 29 **caractérisé par le fait que** l'on ajoute une diamine primaire de préférence, le 1,1-diphényl-1,2-diaminoéthane, le 1,1-bis(4-méthoxyphényl)-2-méthyl-1,2-diaminoéthane, le 1,1-bis(4-méthoxyphényl)-2-isopropyl-1,2-diaminoéthane, 1,1-bis(4-méthoxyphényl)-2-isobutyl-1,2-diamino-éthane.

## Claims

**1.** Process for the asymmetric hydrogenation of a ketonic compound and derivative, **characterized in that** the asymmetric hydrogenation of said compound is carried out in the presence of an effective amount of a metal complex comprising, as the ligand, an optically active diphosphine having one of the following formulae:

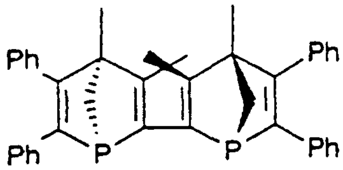

(Ia) - (S,S) - (+)

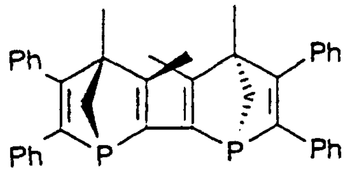

(Ib) - (R,R) - (-)

**2.** Process according to Claim 1, **characterized in that** the metal complex comprises an optically active diphosphine of formula (Ia) or (Ib) and a transition metal selected from rhodium, ruthenium, rhenium, iridium, cobalt, nickel, platinum, palladium.

**3.** Process according to one of Claims 1 and 2, **characterized in that** the metal complex has one of the following formulae:

$$[ML_2(P^*P)]Y \qquad (IIa)$$

$$[ML_2(P^*P)]Y \qquad (IIb)$$

in which:

- (P*P) in formula (IIa) is the diphosphine of formula (Ia) and in formula (IIb) is the diphosphine of formula (Ib);
- M is rhodium or iridium;
- Y is an anionic coordinating ligand;
- L is a neutral ligand.

**4.** Process according to Claim 3, **characterized in that** the complex has formula (IIa) or (IIb) in which:

- L is an olefin having from 2 to 12 carbon atoms and two ligands L can be bonded together to form a polyunsaturated, linear or cyclic hydrocarbon chain, L preferably being 1,5-cyclooctadiene, norbornadiene or ethylene;
- Y is the anion $PF_6^-$, $PCl_6^-$, $BF_4^-$, $BCl_4^-$, $SbF_6^-$, $SbCl_6^-$, $BPh_4^-$, $ClO_4^-$, $CN^-$ or $CF_3SO_3^-$, halogen, preferably $Cl^-$ or $Br^-$, a 1,3-diketonate, alkylcarboxylate or halogenoalkylcarboxylate anion with a lower alkyl radical, or a phenylcarboxylate or phenolate anion whose benzene ring can be substituted by lower alkyl radicals and/or halogen atoms.

**5.** Process according to one of Claims 1 and 2, **characterized in that** the metal complex has one of the following formulae:

$$[IrL(P^*P)]Y \qquad (IIIa)$$

$$[IrL(P^*P)]Y \qquad (IIIb)$$

in which (P*P), L and Y are as defined for formulae (IIa) and (IIb) in Claims 3 and 4.

**6.** Process according to one of Claims 1 and 2, **characterized in that** the metal complex has one of the following formulae:

$$[RuY_1Y_2(P^*P)] \quad (IVa)$$

$$[RuY_1Y_2(P^*P)] \quad (IVb)$$

in which:

- (P*P) in formula (IVa) is the diphosphine of formula (Ia) and in formula (IVb) is the diphosphine of formula (Ib);
- $Y_1$ and $Y_2$, which are identical or different, are preferably the anion $PF_6^-$, $PCl_6^-$, $BF_4^-$, $BCl_4^-$, $SbF_6^-$, $SbCl_6^-$, $BPh_4^-$, $ClO_4^-$ or $CF_3SO_3^-$, a halogen atom, more particularly chlorine or bromine, or a carboxylate anion, preferably acetate or trifluoroacetate.

**7.** Process according to one of Claims 1 and 2, **characterized in that** the metal complex has one of the following formulae:

$$[RuY_1Ar(P^*P)Y_2] \quad (IVc)$$

$$[RuY_1Ar(P^*P)Y_2] \quad (IVd)$$

in which:

- (P*P) in formula (IVc) is the diphosphine of formula (Ia) and in formula (IVd) is the diphosphine of formula (Ib);
- Ar is benzene, p-methylisopropylbenzene or hexamethylbenzene;
- $Y_1$ is a halogen atom, preferably chlorine or bromine;
- $Y_2$ is an anion, preferably $PF_6^-$, $PCl_6^-$, $BF_4^-$, $BCl_4^-$, $SbF_6^-$, $SbCl_6^-$, $BPh_4^-$, $ClO_4^-$ or $CF_3SO_3^-$.

**8.** Process according to one of Claims 1 to 7, **characterized in that** the ketonic compound or derivative has general formula (V):

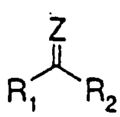 (V)

in which:

- $R_1$ is different from $R_2$;
- $R_1$ and $R_2$ are a hydrocarbon radical having from 1 to 30 carbon atoms and optionally comprising one or more functional groups;
- $R_1$ and $R_2$ can form a ring optionally comprising another heteroatom;
- Z is or comprises an oxygen or nitrogen heteroatom or a functional group comprising at least one of these heteroatoms.

**9.** Process according to Claim 8, **characterized in that** the ketonic compound or derivative has general formula (V) in which the radicals $R_1$ and $R_2$ are a substituted or unsubstituted, monovalent hydrocarbon radical which can be a linear or branched, saturated or unsaturated, acyclic aliphatic radical; a monocyclic or polycyclic, saturated, unsaturated or aromatic carbocyclic or heterocyclic radical.

**10.** Process according to one of Claims 8 and 9, **characterized in that** the ketonic compound or derivative has formula (Va):

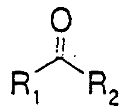

(Va)

in which:

- $R_1$ is different from $R_2$, the radicals $R_1$ and $R_2$ being a hydrocarbon radical having from 1 to 30 carbon atoms and optionally comprising another ketone group and/or an acid, ester, thioacid or thioester group;
- $R_1$ and $R_2$ can form a substituted or unsubstituted carbocyclic or heterocyclic ring having 5 or 6 atoms.

**11.** Process according to Claim 10, **characterized in that** the ketonic compound is a simple ketone.

**12.** Process according to one of Claims 10 and 11, **characterized in that** the ketonic compound has formula ($Va_1$):

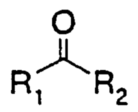

($Va_1$)

in which:

- $R_1$ and $R_2$ are:
  . a linear or branched alkyl radical having from 1 to 12 carbon atoms, preferably from 1 to 4 carbon atoms, such as methyl, ethyl, propyl, isopropyl, butyl, isobutyl, sec-butyl or tert-butyl;
  . an alkenyl radical having from 2 to 12 carbon atoms, preferably an allyl radical;
  . an optionally substituted phenyl, naphthyl or benzyl radical;
  . a triphenylmethyl radical;
  . a radical of the formula

$$-R_3-OH$$

$$-R_3-O-R_4$$

$$-R_3-CO-R_4$$

$$-R_3-COOR_4$$

$$-R_3-CHO$$

$$-R_3-NO_2$$

$$-R_3-CN$$

$$-R_3-N(R_4)_2$$

$-R_3-CO-N(R_4)_2$

$-R_3-PO-(OR_4)_2$

$-R_3-SH$

$-R_3-X$

$-R_3-CF_3$

in which $R_3$ is a valence bond or a saturated or unsaturated, linear or branched, divalent hydrocarbon radical having from 1 to 6 carbon atoms, such as, for example, methylene, ethylene, propylene, isopropylene or isopropylidene; the radicals $R_4$, which are identical or different, are a hydrogen atom, a linear or branched alkyl radical having from 1 to 6 carbon atoms, a benzyl radical or a phenyl radical; X symbolizes a halogen atom, preferably a chlorine, bromine or fluorine atom;

- $R_1$ and $R_2$ can form a substituted or unsubstituted carbocyclic or heterocyclic ring having 5 or 6 atoms.

13. Process according to Claim 12, **characterized in that** the ketonic compound of formula ($Va_1$) is selected from:

- methyl phenyl ketone,
- isopropyl phenyl ketone,
- cyclopropyl phenyl ketone,
- allyl phenyl ketone,
- p-methylphenyl methyl ketone,
- benzyl phenyl ketone,
- phenyl triphenylmethyl ketone,
- o-bromoacetophenone,
- α-bromoacetone,
- α-dibromoacetone,
- α-chloroacetone,
- α-dichloroacetone,
- α-trichloroacetone,
- 1-chloro-3,3-dichloroacetone,
- 1-chloro-2-oxobutane,
- 1-fluoro-2-oxobutane,
- 1-chloro-3-methyl-2-butanone,
- α-chloroacetophenone,
- 1-chloro-3-phenylacetone,
- α-methylaminoacetone,
- α-dimethylaminoacetone,
- 1-butylamino-2-oxopropane,
- 1-dibutylamino-2-oxopropane,
- 1-methylamino-2-oxobutane,
- 1-dimethylamino-2-oxobutane,
- 1-dimethylamino-3-methyl-2-oxobutane,
- 1-dimethylamino-2-oxopentane,
- α-dimethylaminoacetophenone,
- α-hydroxyacetone,
- 1-hydroxy-3-methyl-2-butanone,
- 1-hydroxy-2-oxobutane,
- 1-hydroxy-2-oxopentane,
- 1-hydroxy-2-oxohexane,

- 1-hydroxy-2-oxo-3-methylbutane,
- α-hydroxyacetophenone,
- 1-hydroxy-3-phenylacetone,
- α-methoxyacetone,
- α-methoxyacetophenone,
- α-methoxyacetophenone,
- α-ethoxyacetone,
- α-butoxyacetophenone,
- α-chloro-p-methoxyacetophenone,
- α-naphthenone,
- 1-ethoxy-2-oxobutane,
- 1-butoxy-2-oxobutane,
- α-dimethoxyphosphorylacetone,
- 3-oxotetrahydrothiophene.

**14.** Process according to Claim 10, **characterized in that** the ketonic compound is a diketone.

**15.** Process according to one of Claims 10 and 14, **characterized in that** the ketonic compound has formula ($Va_2$):

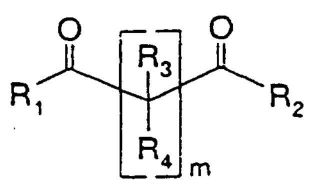

($Va_2$)

in which:

- m is equal to 0, 1, 2 or 3 and is preferably equal to 0 or 1;
- the radicals $R_1$ and $R_2$, which are different, are:

  . a linear or branched alkyl radical having from 1 to 12 carbon atoms, possibly carrying a halogen atom, preferably from 1 to 4 carbon atoms, such as methyl, ethyl, propyl, isopropyl, butyl, isobutyl, sec-butyl or tert-butyl;
  . an alkenyl radical having from 2 to 12 carbon atoms, preferably an allyl radical;
  . an optionally substituted phenyl, naphthyl or benzyl radical;
  . a radical of the formula

$$-R_5-OH$$

$$-R_5-O-R_6$$

$$-R_5-CO-R_6$$

$$-R_5-COOR_6$$

$$-R_5-N(R_6)_2$$

$$-R_5-CO-N(R_6)_2$$

$$-R_5-PO-(OR_6)_2$$

$$-R_5-SH$$

$$-R_5-X$$

$$-R_5-CF_3$$

in which $R_5$ is a valence bond or a saturated or unsaturated, linear or branched, divalent hydrocarbon radical having from 1 to 6 carbon atoms, such as, for example, methylene, ethylene, propylene, isopropylene or isopropylidene; the radicals $R_6$, which are identical or different, are a hydrogen atom, a linear or branched alkyl radical having from 1 to 6 carbon atoms, a benzyl radical or a phenyl radical; X symbolizes a halogen atom, preferably a chlorine, bromine or fluorine atom;

- one of the radicals $R_1$ and $R_2$ can be a hydrogen atom;
- the radicals $R_3$ and $R_4$, which are identical or different, are:
  . a hydrogen atom;
  . a linear or branched alkyl radical having from 1 to 12 carbon atoms, possibly carrying a hydrogen atom, preferably from 1 to 4 carbon atoms, such as methyl, ethyl, propyl, isopropyl, butyl, isobutyl, sec-butyl or tert-butyl;
  . a halogen atom;
  . a group of the type $-R_5-COOR_6$, in which $R_5$ and $R_6$ are as defined above;
- $R_1$ or $R_2$ and $R_3$ or $R_4$ can form a substituted or unsubstituted carbocyclic or heterocyclic ring having 5 or 6 atoms.

**16.** Process according to Claim 15, **characterized in that** the ketonic compound of formula ($Va_2$) is selected from:

- α-formylacetone,
- diacetyl,
- 3,4-dioxohexane,
- 4,5-dioxooctane,
- 1-phenyl-1,2-dioxopropane,
- 1-phenyl-2,3-dioxobutane,
- dibenzoyl,
- p-methoxydibenzoyl,
- 1,2-cyclopentanedione,
- 1,2-cyclohexanedione,
- acetylacetone,
- 3,5-heptanedione,
- 4,6-nonanedione,
- 5,7-undecanedione,
- 2,4-hexanedione,
- 2,4-heptanedione,
- 2,4-octanedione,
- 2,4-nonanedione,
- 3,5-nonanedione,
- 3,5-decanedione,
- 2,4-dodecanedione,
- 1-phenyl-1,3-butanedione,
- 1-phenyl-1,3-pentanedione,
- 1-phenyl-1,3-hexanedione,
- 1-phenyl-1,3-heptanedione,

- 3-methyl-2,4-pentanedione,
- 1,3-diphenyl-1,3-propanedione,
- 1,5-diphenyl-2,4-pentanedione,
- 1,3 -di(trifluoromethyl)-1,3-propanedione,
- 3-chloro-2,4-pentanedione,
- 1,5-dichloro-2,4-pentanedione,
- 1,5-dihydroxy-2,4-pentanedione,
- 1,5-dibenzyloxy-2,4-pentanedione,
- 1,5-diamino-2,4-pentanedione,
- 1,5-di(methylamino)-2,4-pentanedione,
- 1,5-di(dimethylamino)-2,4-pentanedione,
- methyl 3,5-dioxohexanoate,
- 3-carbomethoxy-2,4-pentanedione,
- 3-carboethoxy-2,4-pentanedione,
- 1,3-cyclopentanedione,
- 1,3-cyclohexanedione,
- 1,3-cycloheptanedione,
- 5-carboethoxy-1,3-cyclopentanedione,
- 2-acetyl-1-cyclopentanone,
- 2-acetyl-1-cyclohexanone.

**17.** Process according to Claim 10, **characterized in that** the ketonic compound is a keto acid, keto thioacid or derivative.

**18.** Process according to one of Claims 10 and 17, **characterized in that** the ketonic compound has formula ($Va_3$) or ($Va_4$):

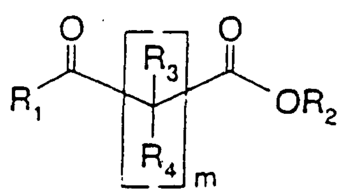 ($Va_3$) or 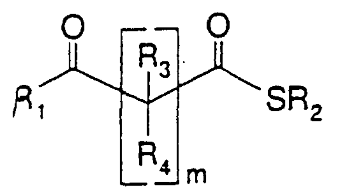 ($Va_4$)

in which:

- m is equal to 0, 1, 2 or 3 and is preferably equal to 0 or 1;
- the radical $R_1$ is:
  - . a linear or branched alkyl radical having from 1 to 12 carbon atoms, possibly carrying a halogen atom, preferably from 1 to 4 carbon atoms, such as methyl, ethyl, propyl, isopropyl, butyl, isobutyl, sec-butyl or tert-butyl;
  - . an alkenyl radical having from 2 to 12 carbon atoms, preferably an allyl radical;
  - . an optionally substituted phenyl, naphthyl or benzyl radical;
  - . a radical of the formula

$$-R_5-OH$$

$$-R_5-O-R_6$$

$$-R_5-CO-R_6$$

$-R_5-COOR_6$

$-R_5-N(R_6)_2$

$-R_5-CO-N(R_6)_2$

$-R_5-PO-(OR_6)_2$

$-R_5-SH$

$-R_5-X$

$-R_5-CF_3$

in which $R_5$ is a valence bond or a saturated or unsaturated, linear or branched, divalent hydrocarbon radical having from 1 to 6 carbon atoms, such as, for example, methylene, ethylene, propylene, isopropylene or isopropylidene; the radicals $R_6$, which are identical or different, are a hydrogen atom or a linear or branched alkyl radical having from 1 to 6 carbon atoms, a benzyl radical or a phenyl radical; and X symbolizes a halogen atom, preferably a chlorine, bromine or fluorine atom;

- the radical $R_2$ is:

  . a hydrogen atom;
  . a linear or branched alkyl radical having from 1 to 6 carbon atoms, preferably from 1 to 4 carbon atoms, such as methyl, ethyl, propyl, isopropyl, butyl, isobutyl, sec-butyl or tert-butyl;
  . a benzyl radical;
  . a phenyl radical;

- the radicals $R_3$ and $R_4$, which are identical or different, are:

  . a hydrogen atom;
  . a linear or branched alkyl radical having from 1 to 12 carbon atoms, preferably from 1 to 4 carbon atoms, such as methyl, ethyl, propyl, isopropyl, butyl, isobutyl, sec-butyl, tert-butyl;
  . a halogen atom;
  . a group of the type $-R_5-COOR_6$, in which $R_5$ and $R_6$ are as defined above;
  . a group of the type $-R_5-CO-N(R_6)_2$, in which $R_5$ and $R_6$ are as defined above;

- $R_1$ and $R_2$ or $R_1$ and $R_3$ or $R_1$ and $R_4$ or $R_2$ and $R_4$ can form a substituted or unsubstituted carbocyclic or heterocyclic ring having 5 or 6 atoms.

**19.** Process according to Claim 18, **characterized in that** the ketonic compound of formula ($Va_3$) or ($Va_4$) is selected from:

- 2-acetylbenzoic acid,
- pyruvic acid,
- 2-oxobutanoic acid,
- 3-methyl-2-oxobutanoic acid,
- phenylglyoxylic acid,
- phenylpyruvic acid,
- p-methoxyphenylpyruvic acid,
- 3,4-dimethoxyphenylpyruvic acid,

- methyl acetoacetate,
- ethyl acetoacetate,
- n-propyl acetoacetate,
- isopropyl acetoacetate,
- n-butyl acetoacetate,
- t-butyl acetoacetate,
- n-pentyl acetoacetate,
- n-hexyl acetoacetate,
- n-heptyl acetoacetate,
- n-octyl acetoacetate,
- methyl 3-oxopentanoate,
- methyl 3-oxohexanoate,
- methyl 3-oxoheptanoate,
- ethyl 3-oxooctanoate,
- ethyl 3-oxononanoate,
- ethyl 3-oxodecanoate,
- ethyl 3-oxoundecanoate,
- ethyl 3-oxo-3-phenylpropanoate,
- ethyl 4-phenyl-3-oxobutanoate,
- methyl 5-phenyl-3-oxopentanoate,
- ethyl 3-oxo-3-p-methoxyphenylpropanoate,
- methyl 4-chloroacetoacetate,
- ethyl 4-chloroacetoacetate,
- methyl 4-fluoroacetoacetate,
- ethyl 3-trifluoromethyl-3-oxopropionate,
- ethyl 4-hydroxy-3-oxobutanoate,
- methyl 4-methoxyacetoacetate,
- methyl 4-tert-butoxyacetoacetate,
- methyl 4-benzyloxy-3-oxobutanoate,
- ethyl 4-benzyloxy-3-oxobutanoate,
- methyl 4-amino-3-oxobutanoate,
- ethyl 3-methylamino-3-oxobutanoate,
- methyl 4-dimethylamino-3-oxobutanoate,
- ethyl 4-dimethylamino-3-oxobutanoate,
- methyl 2-methylacetoacetate,
- ethyl 2-methylacetoacetate,
- ethyl 2-chloroacetoacetate,
- diethyl 2-acetylsuccinate,
- diethyl 2-acetylglutarate,
- dimethyl acetylmalonate,
- thiomethyl acetoacetate,
- thioethyl acetoacetate,
- thiophenyl acetoacetate,
- methyl pyruvate,
- ethyl 3-methyl-2-oxobutanoate,
- ethyl phenylglyoxylate,
- methyl phenylpyruvate,
- ethyl phenylpyruvate,
- 3-oxobutanoic dimethylamide,
- 3-oxobutanoic benzylamide,
- 2-carboethoxycyclopentanone,
- 2-carboethoxycyclohexanone,
- ketopentalactone,
- 4-oxopentanoic acid,
- 4-oxohexanoic acid,
- 4-oxoheptanoic acid,
- 4-oxodecanoic acid,
- 4-oxododecanoic acid,

- 4-phenyl-4-oxybutyric acid,
- 4-p-methoxyphenyl-4-oxybutyric acid,
- 4-(3,4-dimethoxyphenyl)-4-oxobutyric acid,
- 4-(3,4,5-trimethoxyphenyl)-4-oxobutyric acid,
- 4-p-chlorophenyl-4-oxybutyric acid,
- 4-phenyl-4-oxobutyric acid.

**20.** Process according to Claim 10, **characterized in that** the ketonic compound has formula (Va) in which the radicals $R_1$ and $R_2$ form, with the carbon atom carrying the carbonyl group, one or more saturated and/or unsaturated or optionally aromatic rings, preferably two rings, comprising 5 or 6 carbon atoms in each ring and optionally carrying another carbonyl group and/or a substituent.

**21.** Process according to one of Claims 8 and 9, **characterized in that** the ketonic derivative has formula (Vb):

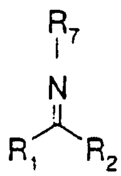
(Vb)

in which:

- $R_1$ and $R_2$, which are different, are as defined above; and
- $R_7$ is:
  - . a hydrogen atom;
  - . a hydroxyl group;
  - . a group $OR_8$;
  - . a hydrocarbon radical $R_8$;
  - . a group of the formula

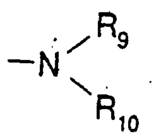

  - . a group of the formula

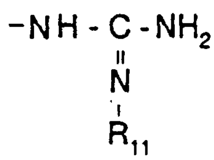

where $R_8$, $R_9$, $R_{10}$ and $R_{11}$ are a hydrogen atom or a hydrocarbon group having from 1 to 30 carbon atoms.

**22.** Process according to Claim 21, **characterized in that** the ketonic derivative has one of the following formulae:

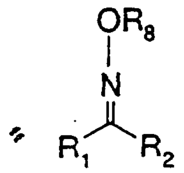
(Vb1)

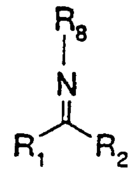
(Vb2)

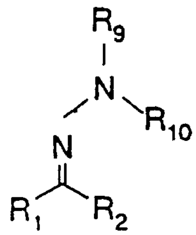
(Vb3)

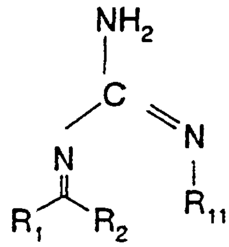
(Vb4)

in which:

- the radicals $R_1$, $R_2$ and $R_8$ to $R_{11}$ are:

  . a linear or branched alkyl radical having from 1 to 12 carbon atoms;
  . a cycloalkyl radical having from 5 to 12 carbon atoms;
  . an aryl radical having from 6 to 12 carbon atoms;
  . an aralkyl radical having from 7 to 12 carbon atoms;
  . an aryl radical having from 6 to 12 carbon atoms carrying substituents such as an alkyl or alkoxy radical having from 1 to 4 carbon atoms, an amino, $(C_1-C_4)$-alkylamino or di$(C_1-C_4)$alkylamino group, a nitro group, a halogen atom or a $(C_1-C_4)$alkoxycarbonyl group;
  . an aryl radical having from 6 to 12 carbon atoms;
  . a saturated or unsaturated heterocyclic radical;
  . an alkanoyl radical having from 1 to 12 carbon atoms;
  . an arylcarbonyl radical having from 6 to 12 carbon atoms;
  . an arylalkanoyl radical having from 6 to 12 carbon atoms;

- $R_1$ and $R_2$, $R_1$ and $R_8$, $R_2$ and $R_8$, $R_1$ and $R_9$, $R_2$ and $R_{10}$, $R_1$ and $R_{11}$, $R_2$ and $R_{11}$ can form a substituted or unsubstituted, monocyclic or polycyclic carbocyclic or heterocyclic ring having 5 or 6 atoms in each ring.

**23.** Process according to one of Claims 21 and 22, **characterized in that** the ketonic derivative has one of formulae ($Vb_1$) to ($Vb_4$) in which the radicals $R_1$ and $R_2$, $R_8$ to $R_{11}$, which are identical or different, are:

. a linear or branched alkyl radical having from 1 to 4 carbon atoms;
. a cyclopentyl or cyclohexyl radical;
. a phenyl radical;
. a benzyl or phenylethyl radical;
. a phenyl radical carrying substituents such as an alkyl or alkoxy radical having from 1 to 4 carbon atoms, an amino, $(C_1-C_4)$alkylamino or di$(C_1-C_4)$alkylamino group, a nitro group, a halogen atom or a $(C_1-C_4)$alkoxycarbonyl group;
. a naphthyl radical;
. a saturated or unsaturated, oxygen-containing or nitrogen-containing heterocyclic radical having 5 or 6 atoms;
. an acetyl radical or a benzoyl radical;
. an arylalkanoyl radical having from 6 to 12 carbon atoms.

**24.** Process according to one of Claims 21 to 23, **characterized in that** the ketonic derivative is selected from:

- acetophenone oxime
- N-isobutyl-2-iminopropane
- N-isobutyl-1-methoxy-2-iminopropane
- N-benzyl- 1-imino-1-(phenyl)ethane

- N-benzyl-1-imino-1-(4-methoxyphenyl)ethane
- N-benzyl-1-imino-1-(2-methoxyphenyl)ethane
- N-phenyl-2-iminopentane
- N-(2,6-dimethylphenyl)-2-iminopentane
- N-(2,4,6-trimethylphenyl)-2-iminopentane
- N-phenyl-1-imino-1-phenylethane
- N-phenyl-1-methoxy-2-iminopropane
- N-(2,6-dimethylphenyl)-1-methoxy-2-iminopropane
- N-(2-methyl-6-ethylphenyl)-1-methoxy-2-iminopropane
- 1-cyclohexyl-1-(2-benzoylhydrazono)ethane,
- 1-phenyl-1-(2-benzoylhydrazono)ethane,
- 1-p-methoxyphenyl-1-(2-benzoylhydrazono)ethane,
- 1-p-ethoxyphenyl-1-(2-benzoylhydrazono)ethane,
- 1-p-nitrophenyl-1-(2-benzoylhydrazono)ethane,
- 1-p-bromophenyl-1-(2-benzoylhydrazono)ethane,
- 1-p-carboethoxyphenyl-1-(2-benzoylhydrazono)ethane,
- 1,2-diphenyl-1-(2-benzoylhydrazono)ethane,
- 3-methyl-2-(2-p-dimethylaminobenzoylhydrazono)butane,
- 1-phenyl-1-(2-p-methoxybenzoylhydrazono)ethane,
- 1-phenyl-1-(2-p-dimethylaminobenzoylhydrazono)ethane,
- ethyl-2-(2-benzoylhydrazono)propionate,
- methyl-2-(2-benzoylhydrazono)butyrate,
- methyl-2-(2-benzoylhydrazono)valerate,
- methyl-2-phenyl-2-(2-benzoylhydrazono)acetate,
- the following cyclic ketoimines with an endocyclic or exocyclic bond:

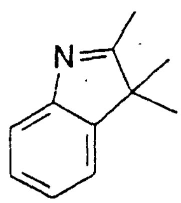
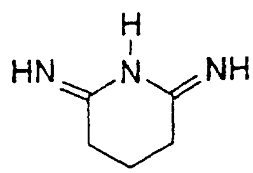
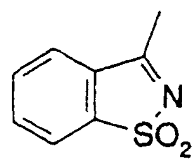

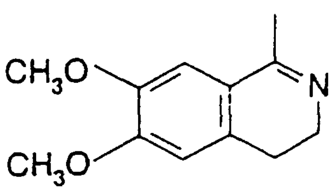
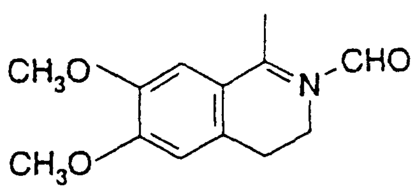
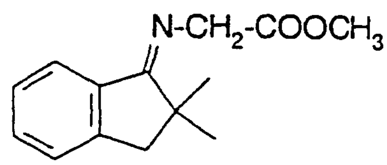

**25.** Process according to one of Claims 1 to 24, **characterized in that** the hydrogenation is generally carried out at a temperature between 20 and 100°C.

**26.** Process according to one of Claims 1 to 25, **characterized in that** the hydrogen pressure can be between 0.1 and 200 bar and more preferably between 1 and 150 bar.

**27.** Process according to one of Claims 1 to 26, **characterized in that** at least one complex according to one of Claims 2 to 7 is used.

**28.** Process according to one of Claims 1 to 27, **characterized in that** the ratio of the number of metal atoms present in the complex to the number of mol of compound to be hydrogenated is between 0.1 and 0.0001.

**29.** Process according to one of Claims 1 to 28, **characterized in that** a basic compound, preferably potassium hydroxide, is added after the formation of the hydrogenation complex.

**30.** Process according to one of Claims 1 to 29, **characterized in that** a primary diamine, preferably 1,1 -diphenyl-1,2-diaminoethane, 1,1-bis(4-methoxyphenyl)-2-methyl-1,2-diaminoethane, 1,1 -bis(4-methoxyphenyl)-2-isopropyl-1,2-diaminoethane, 1,1-bis(4-methoxyphenyl)-2-isobutyl-1,2-diaminoethane, is added.

**Patentansprüche**

**1.** Verfahren zur asymmetrischen Hydrierung einer Ketoverbindung und ihres Derivates, **dadurch gekennzeichnet, daß** man eine asymmetrische Hydrierung dieser Verbindung in Gegenwart einer wirksamen Menge eines Metallkomplexes durchführt, der als Liganden ein optisch aktives Diphosphin umfaßt, welches einer der beiden folgenden Formeln entspricht:

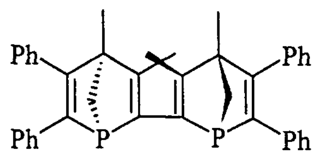

(Ia)-(S,S)-(+)

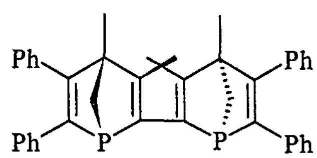

(Ib)-(R,R)-(-)

**2.** Verfahren nach Anspruch 1, **dadurch gekennzeichnet, daß** der Metallkomplex ein optisch aktives Diphosphin der Formel (Ia) oder (Ib) und ein Übergangsmetall umfaßt, das ausgewählt ist aus Rhodium, Ruthenium, Rhenium, Iridium, Kobalt, Nickel, Platin und Palladium.

**3.** Verfahren nach Anspruch 1 oder 2, **dadurch gekennzeichnet, daß** der Metallkomplex einer der beiden folgenden Formeln entspricht:

$$[M\ L_2(P^*P)]Y \qquad (IIa)$$

$$[M\ L_2(P^*P)]Y \qquad (IIb)$$

wobei in den Formeln:

- (P*P) in der Formel (IIa) das Diphosphin der Formel (Ia) und in der Formel (IIb) das Diphosphin der Formel (Ib) darstellt,
- M Rhodium oder Iridium darstellt,
- Y einen anionischen Koordinierungsliganden darstellt und
- L einen neutralen Liganden darstellt.

**4.** Verfahren nach Anspruch 3, **dadurch gekennzeichnet, daß** der Komplex der Formel (IIa) oder (IIb) entspricht, in der:

- L ein Olefin mit 2 bis 12 Kohlenstoffatomen darstellt und zwei Liganden L verbunden sein können, um untereinander eine lineare oder ringförmige, polyungesättigte Kohlenwasserstoffkette zu bilden, wobei L vorzugsweise 1,5-Cyclooctadien, Norbomadien oder Ethylen darstellt,
- Y ein Anion $PF_6^-$, $PCl_6^-$, $BF_4^-$, $BCl_4^-$, $SbF_6^-$, $SbCl_6^-$, $BPh_4^-$, $ClO_4^-$, CN-, $CF_3SO_3^-$, Halogen, vorzugsweise $Cl^-$ oder $Br^-$, ein 1,3-Diketonatanion, Alkylcarboxylat, Haloalkylcarboxylat mit einem niederen Alkylrest oder ein Phenylcarboxylat- oder Phenolatanion, dessen Benzolring durch niedere Alkylreste und/oder Halogenatome substituiert sein kann, darstellt.

**5.** Verfahren nach Anspruch 1 oder 2, **dadurch gekennzeichnet, daß** der Metallkomplex einer der beiden folgenden Formeln entspricht:

$$[Ir\ L\ (P{*}P)]Y \qquad (IIIa)$$

$$[Ir\ L\ (P{*}P)]Y \qquad (IIIb)$$

wobei in den Formel (P*P), L und Y die in den Formeln (IIa) und (IIb) der Ansprüche 3 und 4 angegebenen Bedeutungen haben.

**6.** Verfahren nach Anspruch 1 oder 2, **dadurch gekennzeichnet, daß** der Metallkomplex einer der beiden folgenden Formeln entspricht:

$$[RuY_1Y_2(P{*}P)] \qquad (IVa)$$

$$[RuY_1Y_2(P{*}P)] \qquad (IVb)$$

wobei in den Formeln

- (P*P) in der Formel (IVa) das Diphosphin der Formel (Ia) und in der Formel (IVb) das Diphosphin der Formel (Ib) darstellt,
- $Y_1$ und $Y_2$, identisch oder verschieden, vorzugsweise ein Anion $PF_6^-$, $PCl_6^-$, $BF_4^-$, $BCl_4^-$, $SbF_6^-$, $SbCl_6^-$, $BPh_4^-$, $ClO_4^-$, $CF_3SO_3^-$, ein Halogenatom, insbesondere Chlor oder Brom, oder ein Carboxylatanion, vorzugsweise Acetat oder Trifluoracetat, darstellen.

**7.** Verfahren nach Anspruch 1 oder 2, **dadurch gekennzeichnet, daß** der Metallkomplex einer der beiden folgenden Formeln entspricht:

$$[RuY_1Ar(P{*}P)Y_2] \qquad (IVc)$$

$$[RuY_1Ar(P{*}P)Y_2] \qquad (IVd)$$

wobei in den Formeln

- (P*P) in der Formel (IVc) das Diphosphin der Formel (Ia) und in der Formel (IVd) das Diphosphin der Formel (Ib) darstellt,
- Ar Benzol, p-Methylisopropylbenzol oder Hexamethylbenzol darstellt,
- $Y_1$ ein Halogenatom, vorzugsweise Chlor oder Brom, darstellt,
- $Y_2$ ein Anion darstellt, vorzugsweise $PF_6^-$, $PCl_6^-$, $BF_4^-$, $BCl_4^-$, $SbF_6^-$, $SbCl_6^-$, $BPh_4^-$, $ClO_4^-$ oder $CF_3SO_3^-$.

**8.** Verfahren nach einem der Ansprüche 1 bis 7, **dadurch gekennzeichnet, daß** die Ketoverbindung oder ihr Derivat der allgemeinen Formel (IV) entspricht

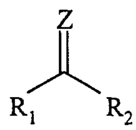

(V)

wobei in der Formel (V)

- $R_1$ verschieden von $R_2$ ist,
- $R_1$ und $R_2$ einen Kohlenwasserstoffrest mit 1 bis 30 Kohlenstoffatomen darstellen, der gegebenenfalls ein oder mehrere funktionelle Gruppen umfaßt,
- $R_1$ und $R_2$ einen Ring bilden können, der gegebenenfalls ein weiteres Heteroatom umfaßt,
- Z ein Heteroatom, Sauerstoff oder Stickstoff, oder eine funktionelle Gruppe, die mindestens eines dieser Heteroatome umfaßt, ist oder umfaßt.

**9.** Verfahren nach Anspruch 8, **dadurch gekennzeichnet, daß** die Ketoverbindung oder ihr Derivat der allgemeinen Formel (V) entspricht, in der die Reste $R_1$ und $R_2$ einen gegebenenfalls substituierten, monovalenten (einwertigen) Kohlenwasserstoffrest, der ein linearer oder verzweigter, gesättigter oder ungesättigter, acyclischer aliphatischer Rest sein kann, oder einen polycyclischen oder monocyclischen, gesättigten, ungesättigten oder aromatischen, heterocyclischen oder carbocyclischen Rest darstellen.

**10.** Verfahren nach Anspruch 8 oder 9, **dadurch gekennzeichnet, daß** die Ketoverbindung oder ihr Derivat der allgemeinen Formel (Va) entspricht:

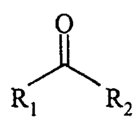

(Va)

wobei in der Formel (Va)

- $R_1$ von $R_2$ verschieden ist, wobei die Reste $R_1$ und $R_2$ einen Kohlenwasserstoffrest mit 1 bis 30 Kohlenstoffatomen darstellen, der gegebenenfalls eine weitere Ketofunktion und/oder eine Säure-, Ester-, Thiosäureoder Thioesterfunktion umfaßt,
- $R_1$ und $R_2$ einen gegebenenfalls substituierten, heterocyclischen oder carbocyclischen Ring mit 5 bis 6 Atomen bilden können.

**11.** Verfahren nach Anspruch 10, **dadurch gekennzeichnet, daß** die Ketoverbindung ein einfaches Keton ist.

**12.** Verfahren nach Anspruch 10 oder 11, **dadurch gekennzeichnet, daß** die Ketoverbindung der allgemeinen Formel ($Va_1$) entspricht.

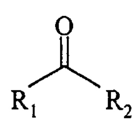

($Va_1$)

wobei in der Formel ($Va_1$)

- $R_1$ und $R_2$ darstellen:

  - einen linearen oder verzweigten Alkylrest mit 1 bis 12 Kohlenstoffatomen, vorzugsweise 1 bis 4 Kohlenstoffatomen, wie Methyl, Ethyl, Propyl, Isopropyl, Butyl, Isobutyl, sek.-Butyl, tert.-Butyl,
  - einen Alkenylrest mit 2 bis 12 Kohlenstoffatomen, vorzugsweise einen Allylrest,
  - einen gegebenenfalls substituierten Phenyl-, Naphthyl- oder Benzylrest,
  - einen Triphenylmethylrest,
  - einen Rest der Formel:

$$-R_3\text{-OH}$$

$$-R_3\text{-O-}R_4$$

$$-R_3\text{-CO-}R_4$$

$$-R_3\text{-COO}R_4$$

$$-R_3\text{-CHO}$$

$$-R_3\text{-NO}_2$$

$$-R_3\text{-CN}$$

$$-R_3\text{-N}(R_4)_2$$

$$-R_3\text{-CO-N}(R_4)_2$$

$$-R_3\text{-PO-(O}R_4)_2$$

$$-R_3\text{-SH}$$

$$-R_3X$$

$$-R_3CF_3$$

  wobei in den Formeln $R_3$ eine Valenzbindung oder einen linearen oder verzweigten, gesättigten oder ungesättigten, divalenten (zweiwertigen) Kohlenwasserstoffrest mit 1 bis 6 Kohlenstoffatomen, wie beispielsweise Methylen, Ethylen, Propylen, Isopropylen oder Isopropyliden, darstellt; die Reste $R_4$, identisch oder verschieden, ein Wasserstoffatom oder einen linearen oder verzweigten Alkylrest mit 1 bis 6 Kohlenstoffatomen, einen Benzylrest oder einen Phenylrest darstellen; X eine Halogenatom, vorzugsweise ein Chlor-, Brom- oder Fluoratom, darstellt,

- $R_1$ und $R_2$ einen gegebenenfalls substituierten, heterocyclischen oder carbocyclischen Ring mit 5 bis 6 Kohlenstoffatomen bilden können.

**13.** Verfahren nach Anspruch 12, **dadurch gekennzeichnet, daß** die Ketoverbindung der Formel ($Va_1$) ausgewählt ist aus den folgenden Verbindungen:

- Methylphenylketon,
- Isopropylphenylketon,
- Cyclopropylphenylketon,
- Allylphenylketon,
- p-Methylphenylmethylketon,
- Benzylphenylketon,
- Phenyltriphenylmethylketon,
- o-Bromacetophenon,
- α-Bromaceton,
- α-Dibromaceton,
- α-Chloraceton,
- α-Dichloraceton,
- α-Trichloraceton,
- 1-Chlor-3,3-dichloraceton,
- 1-Chlor-2-oxobutan,
- 1-Fluor-2-oxobutan,
- 1-Chlor-3-methyl-2-butanon,
- α-Chloracetophenon,
- 1-Chlor-3-phenylaceton,
- α-Methylaminoaceton,
- α-Dimethylaminoaceton,
- 1-Butylamino-2-oxopropan,
- 1-Dibutylamino-2-oxopropan,
- 1-Methylamino-2-oxobutan,
- 1-Dimethylamino-2-oxobutan,
- 1-Dimethylamino-3-methyl-2-oxobutan,
- 1-Dimethylamino-2-oxopentan,
- α-Dimethylaminoacetophenon,
- α-Hydroxyaceton,
- 1-Hydroxy-3-methyl-2-butanon,
- 1-Hydroxy-2-oxobutan,
- 1-Hydroxy-2-oxopentan,
- 1-Hydroxy-2-oxohexan,
- 1-Hydroxy-2-oxo-3-methylbutan,
- α-Hydroxyacetophenon,
- 1-Hydroxy-3-phenylaceton,
- α-Methoxyaceton,
- α-Methoxyacetophenon,
- α-Methoxyacetophenon,
- α-Ethoxyaceton,
- α-Butoxyacetophenon,
- α-Chlor-p-methoxyacetophenon,
- α-Naphthenon,
- 1-Ethoxy-2-oxobutan,
- 1-Butoxy-2-oxobutan,
- 1-Butoxy-2-oxobutan,
- α-Dimethoxyphosphorylaceton,
- 3-Oxotetrahydrothiophen.

**14.** Verfahren nach Anspruch 10, **dadurch gekennzeichnet, daß** die Ketoverbindung ein Diketon ist.

**15.** Verfahren nach einem der Ansprüche 10 bis 14, **dadurch gekennzeichnet, daß** die Ketoverbindung der allgemeinen Formel ($Va_2$) entspricht

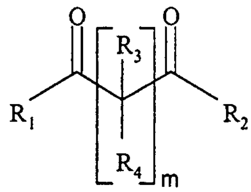

(Va$_2$)

wobei in der Formel (Va$_2$)

- m 0, 1, 2 oder 3, vorzugsweise 0 oder 1, ist,
- die Reste $R_1$ und $R_2$ verschieden sind und die folgenden Reste darstellen:

  - einen linearen oder verzweigten Alkylrest mit 1 bis 12 Kohlenstoffatomen, der gegebenenfalls ein Halogenatom trägt, vorzugsweise mit 1 bis 4 Kohlenstoffatomen, wie Methyl, Ethyl, Propyl, Isopropyl, Butyl, Isobutyl, sek.-Butyl oder tert.-Butyl,
  - einen Alkenylrest mit 2 bis 12 Kohlenstoffatomen, vorzugsweise einen Allylrest,
  - einen gegebenenfalls substituierten Phenyl-, Naphthyl- oder Benzylrest,
  - einen Rest der Formel:

$$- R_5\text{-OH}$$

$$- R_5\text{-O-}R_6$$

$$- R_5\text{-CO-}R_6$$

$$- R_5\text{-COO}R_6$$

$$-R_5\text{-N}(R_6)_2$$

$$- R_5\text{-CO-N}(R_6)_2$$

$$-R_5\text{-PO-(O}R_6)_2$$

$$- R_5\text{-SH}$$

$$- R_5\text{-X}$$

$$- R_5\text{-CF}_3$$

wobei in diesen Formeln $R_5$ eine Valenzbindung oder einen linearen oder verzweigten, gesättigten oder ungesättigten, divalenten (zweiwertigen) Kohlenwasserstoffrest mit 1 bis 6 Kohlenstoffatomen, wie beispielsweise Methylen, Ethylen, Propylen, Isopropylen oder Isopropyliden, darstellt; die Reste $R_6$, identisch oder verschieden, ein Wasserstoffatom oder einen linearen oder verzweigten Alkylrest mit 1 bis 6 Kohlenstoffatomen, einen Benzylrest oder einen Phenylrest darstellen; X ein Halogenatom, vorzugsweise ein Chlor-, Brom- oder Fluoratom, darstellt,

- wobei einer der Reste $R_1$ oder $R_2$ ein Wasserstoffatom darstellen kann,

- die Reste $R_3$ und $R_4$, identisch oder verschieden, darstellen:

  - ein Wasserstoffatom
  - einen linearen oder verzweigten Alkylrest mit 1 bis 12 Kohlenstoffatomen, der gegebenenfalls ein Halogenatom tragen kann, vorzugsweise mit 1 bis 4 Kohlenstoffatomen, wie Methyl, Ethyl, Propyl, Isopropyl, Butyl, Isobutyl, sek.-Butyl oder tert.-Butyl,
  - ein Halogenatom,
  - eine Gruppe vom Typ -$R_5$-COO$R_6$, in der $R_5$ und $R_6$ die zuvor angegebene Bedeutung haben,

- $R_1$ oder $R_2$ und $R_3$ oder $R_4$ einen gegebenenfalls substituierten, heterocyclischen oder carbocyclischen Ring mit 5 bis 6 Kohlenstoffatomen bilden können.

**16.** Verfahren nach Anspruch 15, **dadurch gekennzeichnet, daß** die Ketoverbindung der Formel ($Va_2$) ausgewählt ist aus den folgenden Verbindungen:

- α-Formylaceton,
- Diacetyl,
- 3,4-Dioxohexan,
- 4,5-Dioxohexan,
- 1-Phenyl-1,2-dioxopropan,
- 1-Phenyl-2,3-dioxobutan,
- Dibenzoyl,
- p-Methoxydibenzoyl
- 1,2-Cyclopentandion,
- 1,2-Cyclohexandion,
- Acetylaceton,
- 3,5-Heptandion,
- 4,6-Nonandion,
- 5,7-Undekandion,
- 2,4-Hexandion,
- 2,4-Heptandion,
- 2,4-Oktandion,
- 2,4-Nonandion,
- 3,5-Nonandion,
- 3,5-Dekandion,
- 2,4-Dodekandion,
- 1-Phenyl-1,3-butandion,
- 1-Phenyl-1,3-pentandion,
- 1-Phenyl-1,3-hexandion,
- 1-Phenyl-1,3-heptandion,
- 3-Methyl-2,4-pentandion,
- 1,3-Diphenyl-1,3-propandion,
- 1,5-Diphenyl-2,4-pentandion,
- 1 ,3-Di(trifluormethyl)-1,3-propandion,
- 3-Chlor-2,4-pentandion,
- 1,5-Dichlor-2,4-pentandion,
- 1,5-Dihydroxy-2,4-pentandion,
- 1,5-Dibenzyloxy-2,4-pentandion,
- 1,5-Diamino-2,4-pentandion,
- 1,5-Di(methylamino)-2,4-pentandion,
- 1,5-Di(dimethylamino)-2,4-pentandion,
- 3,5-Dioxomethylhexanoat (3,5-Dioxohexansäuremethylester),
- 3-Carbomethoxy-2,4-pentandion,
- 3-Carboethoxy-2,4-pentandion,

- 1,3-Cyclopentandion,
- 1,3-Cyclohexandion,
- 1,3-Cycloheptandion,
- 5-Carboethoxy-1,3-cyclopentandion,
- 2-Acetyl-1-cyclopentanon,
- 2-Acetyl-1-cyclohexanon.

**17.** Verfahren nach Anspruch 10, **dadurch gekennzeichnet, daß** die Ketoverbindung eine Ketosäure, eine Ketothiosäure oder deren Derivat ist.

**18.** Verfahren nach einem der Ansprüche 10 bis 17, **dadurch gekennzeichnet, daß** die Ketoverbindung der Formel ($Va_3$) oder ($Va_4$) entspricht

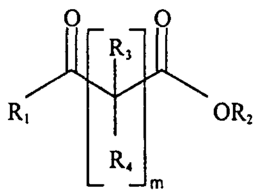

($Va_3$) oder

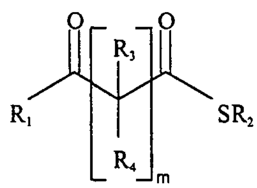

($Va_4$)

wobei in den Formeln ($Va_3$) oder ($Va_4$):

- m 0,1, 2 oder 3, vorzugsweise 0 oder 1, ist,
- der Rest $R_1$ darstellt:
    - einen linearen oder verzweigten Alkylrest mit 1 bis 12 Kohlenstoffatomen, der gegebenenfalls ein Halogenatom trägt, vorzugsweise mit 1 bis 4 Kohlenstoffatomen, wie Methyl, Ethyl, Propyl, Isopropyl, Butyl, Isobutyl, sek.-Butyl oder tert.-Butyl,
    - einen Alkenylrest mit 2 bis 12 Kohlenstoffatomen, vorzugsweise einen Allylrest,
    - einen gegebenenfalls substituierten Phenyl-, Naphthyl- oder Benzylrest,
    - einen Rest der Formel:

$$-R_5-OH$$

$$-R_5-O-R_6$$

$$-R_5-CO-R_6$$

$$-R_5-COOR_6$$

$$-R_5-N(R_6)_2$$

$$-R_5-CO-N(R_6)_2$$

$$-R_5-PO-(OR_6)_2$$

$$-R_5-SH$$

- $R_5$-X

- $R_5$-$CF_3$

wobei in diesen Formeln $R_5$ eine Valenzbindung oder einen linearen oder verzweigten, gesättigten oder ungesättigten, divalenten (zweiwertigen) Kohlenwasserstoffrest mit 1 bis 6 Kohlenstoffatomen, wie beispielsweise Methylen, Ethylen, Propylen, Isopropylen oder Isopropyliden, darstellt; die Reste $R_6$, identisch oder verschieden, ein Wasserstoffatom oder einen linearen oder verzweigten Alkylrest mit 1 bis 6 Kohlenstoffatomen, einen Benzylrest oder einen Phenylrest darstellen; X ein Halogenatom, vorzugsweise ein Chlor-, Brom- oder Fluoratom, darstellt,

- der Rest $R_2$ darstellt:
  - ein Wasserstoffatom,
  - einen linearen oder verzweigten Alkylrest mit 1 bis 6 Kohlenstoffatomen, vorzugsweise mit 1 bis 4 Kohlenstoffatomen, wie Methyl, Ethyl, Propyl, Isopropyl, Butyl, Isobutyl, sek.-Butyl oder tert.-Butyl,
  - einen Benzylrest,
  - einen Phenylrest,
- die Reste $R_3$ und $R_4$, identisch oder verschieden, darstellen:
  - ein Wasserstoffatom,
  - einen linearen oder verzweigten Alkylrest mit 1 bis 12 Kohlenstoffatomen, vorzugsweise mit 1 bis 4 Kohlenstoffatomen, wie Methyl, Ethyl, Propyl, Isopropyl, Butyl, Isobutyl, sek.-Butyl oder tert.-Butyl,
  - ein Halogenatom,
  - eine Gruppe vom Typ -$R_5$-$COOR_6$, in der $R_5$ und $R_6$ die zuvor angegebene Bedeutung haben,
  - eine Gruppe vom Typ -$R_5$-CO-N$(R_6)_2$, in der $R_5$ und $R_6$ die zuvor angegebene Bedeutung haben,
- $R_1$ und $R_2$ oder $R_1$ und $R_3$ oder $R_1$ und $R_4$ oder $R_2$ und $R_4$ einen gegebenenfalls substituierten, heterocyclischen oder carbocyclischen Rest mit 5 bis 6 Kohlenstoffatomen darstellen.

**19.** Verfahren nach Anspruch 18, **dadurch gekennzeichnet, daß** die Ketoverbindung der Formel ($Va_3$) oder ($Va_4$) ausgewählt ist aus den folgenden Verbindungen:

- 2-Acetylbenzoesäure,
- Brenztraubensäure,
- 2-Oxobutansäure,
- 3-Methyl-2-oxobutansäure,
- Phenylglyoxalsäure,
- Phenylbrenztraubensäure,
- p-Methoxyphenylbrenztraubensäure,
- 3,4-Dimethoxyphenylbrenztraubensäure,
- Methylacetoacetat,
- Ethylacetoacetat,
- n-Propylacetoacetat,
- Isopropylacetoacetat,
- n-Butylacetoacetat,
- t-Butylacetoacetat,
- n-Pentylacetoacetat,
- n-Hexylacetoacetat,
- n-Heptylacetoacetat,
- n-Octylacetoacetat,
- Methyl-3-oxopentanoat,
- Methyl-3-oxohexanoat,
- Methyl-3-oxoheptanoat,
- Ethyl-3-oxooctanoat,

- Ethyl-3-oxononanoat,
- Ethyl-3-oxodekanoat,
- Ethyl-3-oxoundekanoat,
- Ethyl-3-oxo-3-phenylpropanoat,
- Ethyl-4-phenyl-3-oxobutanoat,
- Methyl-5-phenyl-3-oxopentanoat,
- Ethyl-3-oxo-3-p-methoxyphenylpropanoat,
- Methyl-4-chloracetoacetat,
- Ethyl-4-chloracetoacetat,
- Methyl-4-fluoracetoacetat,
- Ethyl-3-trifluormethyl-3-oxopropanoat,
- Ethyl-4-hydroxy-3-oxobutanoat,
- Methyl-4-methoxyacetoacetat,
- Methyl-4-tert.-butoxyacetoacetat,
- Methyl-4-benzyloxy-3-oxobutanoat,
- Ethyl-4-benzyloxy-3-oxobutanoat,
- Methyl-4-amino-3-oxobutanoat,
- Ethyl-3-methylamino-3-oxobutanoat,
- Methyl-4-dimethylamino-3-oxobutanoat,
- Ethyl-4-dimethylamino-3-oxobutanoat,
- Methyl-2-methylacetoacetat,
- Ethyl-2-methylacetoacetat,
- Ethyl-2-chloracetoacetat,
- Diethyl-2-acetylsuccinat,
- Diethyl-2-acetylglutarat,
- Dimethylacetylmalonat,
- Thiomethylacetoacetat,
- Thioethylacetoacetat,
- Thiophenylacetoacetat,
- Brenztraubensäuremethylester,
- Ethyl-3-methyl-2-oxobutanoat,
- Ethylphenylglyoxolat,
- Phenylbrenztraubensäuremethylester,
- Phenylbrenztraubensäureethylester,
- Dimethylamid-3-oxobutansäure,
- Benzylamid-3-oxobutansäure,
- 2-Carboethoxycyclopentanon,
- 2-Carboethoxycyclohexanon,
- Ketopentalacton,
- 4-Oxopentansäure,
- 4-Oxohexansäure,
- 4-Oxoheptansäure,
- 4-Oxodekansäure,
- 4-Oxododekansäure,
- 4-Phenyl-4-oxybuttersäure,
- 4-p-Methoxyphenyl-4-oxybuttersäure,
- 4-(3,4-Dimethoxyphenyl)-4-oxobuttersäure,
- 4-(3,4,5-Trimethoxyphenyl)-4-oxobuttersäure,
- 4-p-Chlorphenyl-4-oxybuttersäure,
- 4-Phenyl-4-oxobuttersäure.

**20.** Verfahren nach Anspruch 10, **dadurch gekennzeichnet, daß** die Ketoverbindung der Formel (Va) entspricht, in der die Reste $R_1$ und $R_2$ mit dem Kohlenstoffatom, welches die Carbonylgruppe trägt, einen odere mehrere, vorzugsweise zwei, gesättigte und/oder ungesättigte, gegebenenfalls aromatische Ringe mit 5 bis 6 Kohlenstoffatomen in jedem Ring und gegebenenfalls einer weiteren Carbonylgruppe und/oder einem Substituenten bilden.

**21.** Verfahren nach Anspruch 8 oder 9, **dadurch gekennzeichnet, daß** das Ketoderivat der Formel (Vb) entspricht

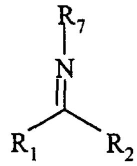

(Vb)

wobei in der Formel (Vb):

- $R_1$ von $R_2$ verschieden ist und $R_1$ und $R_2$ die zuvor angegebene Bedeutung haben,
- $R_7$ darstellt:

  - ein Wasserstoffatom,
  - eine Hydroxylgruppe,
  - eine Gruppe $OR_8$,
  - einen Kohlenwasserstoffrest $R_8$,
  - eine Gruppe der Formel

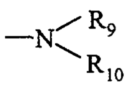

  - eine Gruppe der Formel

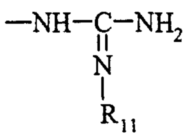

wobei $R_8$, $R_9$, $R_{10}$ und $R_{11}$ ein Wasserstoffatom oder eine Kohlenwasserstoffgruppe mit 1 bis 30 Kohlenstoffatomen darstellen.

**22.** Verfahren nach Anspruch 21, **dadurch gekennzeichnet, daß** das Ketoderivat einer der folgenden Formeln entspricht

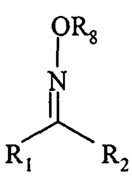

$(Vb_1)$

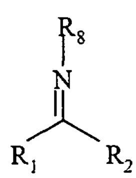

$(Vb_2)$

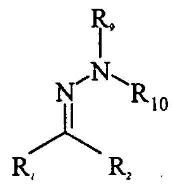

(Vb₃)

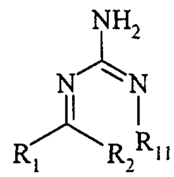

(Vb₄)

wobei in den Formeln ($Vb_1$) bis ($Vb_4$):

- die Reste $R_1$ und $R_2$, $R_8$ bis $R_{11}$ darstellen:

  - einen linearen oder verzweigten Alkylrest mit 1 bis 12 Kohlenstoffatomen,
  - einen Cycloalkylrest mit 5 bis 12 Kohlenstoffatomen,
  - einen Arylrest mit 6 bis 12 Kohlenstoffatomen,
  - einen Aralkylrest mit 7 bis 12 Kohlenstoffatomen,
  - einen Arylrest mit 6 bis 12 Kohlenstoffatomen, die Substituenten tragen können, wie ein Alkyl- oder Alkoxyrest mit 1 bis 4 Kohlenstoffatomen, eine Aminogruppe, eine ($C_1$-$C_4$)-Alkylaminogruppe oder einen Di-($C_1$-$C_4$)-Alkylaminogruppe, eine Nitrogruppe, ein Halogenatom oder eine ($C_1$-$C_4$)-Alkoxycarbonylgruppe,
  - einen Arylrest mit 6 bis 12 Kohlenstoffatomen,
  - einen gesättigten oder ungesättigten heterocyclischen Rest,
  - einen Alkylrest mit 1 bis 12 Kohlenstoffatomen,
  - einen Arylcarbonylrest mit 6 bis 12 Kohlenstoffatomen,
  - einen Arylalkylrest mit 6 bis 12 Kohlenstoffatomen,

- $R_1$ und $R_2$, $R_1$ und $R_8$, $R_2$ und $R_8$, $R_1$ und $R_9$, $R_2$ und $R_{10}$, $R_1$ und $R_{11}$, $R_2$ und $R_{11}$ einen gegebenenfalls substituierten, monocyclischen oder polycyclischen, heterocyclischen oder carbocyclischen Ring mit 5 bis 6 Atomen in jedem Ring bilden können.

**23.** Verfahren nach Anspruch 21 oder 22, **dadurch gekennzeichnet, daß** das Ketoderivat einer der Formeln ($Vb_1$) bis ($Vb_4$) entspricht, in den die Reste $R_1$ und $R_2$, $R_8$ bis $R_{11}$, identisch oder verschieden, darstellen:

- einen linearen oder verzweigten Alkylrest mit 1 bis 4 Kohlenstoffatomen,
- einen Cyclopentyl- oder Cyclohexylrest,
- einen Phenylrest,
- einen Benzyl- oder Phenylethylrest,
- einen Phenylrest, der Substituenten trägt, wie z. B. einen Alkyloder Alkoxyrest mit 1 bis 4 Kohlenstoffatomen, eine Aminogruppe, eine ($C_1$-$C_4$)-Alkylaminogruppe oder eine Di-($C_1$-$C_4$)-Alkylaminogruppe, eine Nitrogruppe, ein Halogenatom oder eine ($C_1$-$C_4$)-Alkoxycarbonylgruppe,
- einen Naphthylrest,
- einen gesättigten oder ungesättigten, sauerstoffhaltigen oder stickstoffhaltigen, heterocyclischen Rest mit 5 oder 6 Atomen,
- einen Acetyl- oder einen Benzylrest,
- einen Arylalkylrest mit 6 bis 12 Kohlenstoffatomen.

**24.** Verfahren nach einem der Ansprüche 21 bis 23, **dadurch gekennzeichnet, daß** das Ketoderivat ausgewählt ist aus den folgenden Verbindungen:

- Acetophenonoxim,
- N-Isobutyl-2-iminopropan,
- N-Isobutyl-1-methoxy-2-iminopropan,
- N-Benzyl-1-imino-1-(phenyl)ethan,
- N-Benzyl-1-imino-1-(4-methoxyphenyl)ethan,

- N-Benzyl-1-imino-1-(2-methoxyphenyl)ethan,
- N-Phenyl-2-iminopentan,
- N-(2,6-Dimethylphenyl)-2-iminopentan,
- N-(2,4,6-Trimethylphenyl)-2-iminopentan,
- N-Phenyl-1-imino-1-phenylethan,
- N-Phenyl-1-methoxy-2-iminopropan,
- N-(2,6-Dimethylphenyl)-1-methoxy-2-iminopropan,
- N-(2-Methyl-6-ethylphenyl)-1-methoxy-2-iminopropan,
- 1-Cyclohexyl-1-(2-benzylhydrazon)ethan,
- 1-Phenyl-1-(2-benzylhydrazon)ethan,
- 1-p-Methoxyphenyl-1-(2-benzylhydrazon)ethan,
- 1-p-Ethoxyphenyl-1-(2-benzylhydrazon)ethan,
- 1-p-Nitrophenyl-1-(2-benzylhydrazon)ethan,
- 1-p-Bromphenyl-1-(2-benzylhydrazon)ethan,
- 1-p-Carboethoxyphenyl-1-(2-benzylhydrazon)ethan,
- 1,2-Diphenyl-1-(2-benzylhydrazon)ethan,
- 1,2-Diphenyl-1-(2-benzylhydrazon)ethan,
- 3-Methyl-2-(2-p-dimethylaminobenzylhydrazon)butan,
- 1-Phenyl-1-(2-p-methoxybenzylhydrazon)ethan,
- 1-Phenyl-1-(2-p-dimethylaminobenzylhydrazon)ethan,
- Ethyl-2-(2-benzylhydrazon)propionat,
- Methyl-2-(2-benzylhydrazon)butyrat,
- Methyl-2-(2-benzylhydrazon)valerat,
- Methyl-2-phenyl-2-(2-benzylhydrazon)acetat,
- ringförmige Ketoimine mit endo- oder exocyclischer Bindung:

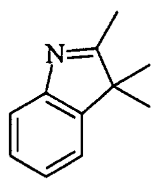
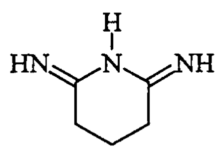
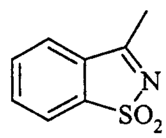

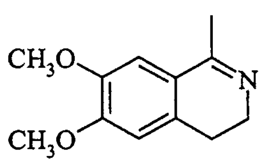
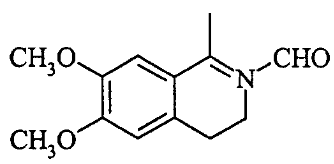
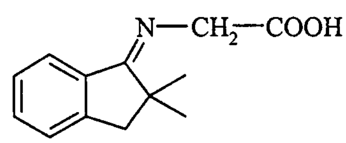

**25.** Verfahren nach einem der Ansprüche 1 bis 24, **dadurch gekennzeichnet, daß** die Hydrierung im allgemeinen bei einer Temperatur zwischen 20 und 100 °C durchgeführt wird.

**26.** Verfahren nach einem der Ansprüche 1 bis 25, **dadurch gekennzeichnet, daß** der Wasserstoffdruck zwischen 0,1 und 200 bar, vorzugsweise zwischen 1 und 150 bar, liegen kann.

**27.** Verfahren nach einem der Ansprüche 1 bis 26, **dadurch gekennzeichnet, daß** man mindestens einen Komplex nach einem der Ansprüche 2 bis 7 verwendet.

**28.** Verfahren nach einem der Ansprüche 1 bis 27, **dadurch gekennzeichnet, daß** das Verhältnis zwischen der Anzahl der in dem Komplex vorhandenen Metallatome und der Anzahl an Mol der zu hydrierenden Verbindung zwischen 0,1 und 0,0001 liegt.

**29.** Verfahren nach einem der Ansprüche 1 bis 28, **dadurch gekennzeichnet, daß** man nach Bildung des Hydrierungskomplexes eine basische Verbindung, vorzugsweise Kaliumcarbonat, hinzugibt.

**30.** Verfahren nach einem der Ansprüche 1 bis 29, **dadurch gekennzeichnet, daß** man ein primäres Amin hinzugibt, vorzugsweise 1,1-Diphenyl-1,2-diaminoethan, 1,1-Bis(4-methoxyphenyl)-2-methyl-1,2-diaminoethan, 1,1-Bis (4-methoxyphenyl)-2-isopropyl-1,2-diaminoethan oder 1,1-Bis(4-methoxyphenyl)-2-isobutyl-1,2-diaminoethan.